# EUROPEAN PATENT SPECIFICATION

(11) **EP 2 620 212 B1**
(45) Date of publication and mention of the grant of the patent: **29.07.2026**
(21) Application number: 13161427.3
(22) Date of filing: 20.07.2007
(51) Int. Cl.: B01J 21/04, B01J 37/26, C07D 301/10, B01J 23/66, B01J 35/60, C04B 35/10

(54) **Method for modifying the surface composition of alpha-alumina**
Verfahren zur Modifizierung der Oberflächenzusammensetzung von alpha-Aluminiumoxid
Procédé de modification de la composition de surface de l'alumine alpha

(30) Priority: 01.11.2006 US 855898 P
(43) Date of publication of application: 31.07.2013
(62) Divisional of application: 07810638.2
(73) Proprietor: Dow Global Technologies LLC, Midland, MI 48674 (US)
(72) Inventor: Wallin, Sten, Midland, MI Michigan 48642 (US); Serafin, Juliana, G., Charleston, WV West Virginia 25311 (US); Bhasin, Madan, M., Charleston, WV West Virginia 25314 (US); Lakso, Steven, R., Sanford, MI Michigan 48657 (US); Howard, Kevin, E., Midland, MI Michigan 48642 (US); Lebaron, Peter, C., Sanford, MI Michigan 48628 (US)
(74) Representative: Beck Greener LLP

(56) References cited:
- WO-A1-2006/091478
- US-A- 4 994 588
- US-A- 6 011 163
- US-A1- 2004 215 026

## Description

### FIELD OF THE INVENTION

This invention relates to a method for modifying the surface composition of alpha-alumina.

### BACKGROUND OF THE INVENTION

Shaped porous bodies comprising alpha-alumina find uses in many areas including catalysis, filtration and separations. Often in these applications, specific properties, such as porosity, total pore volume, pore size, specific surface area, and surface chemistry of the shaped porous body are desired. The shape of the bodies can be constrained by the mechanical properties of the body; a stronger body permits the performance to be optimized while meeting the mechanical requirements of the application. In catalytic applications, both simple shaped bodies such as pellets, rings and saddles and complex shaped bodies such as honeycombs are employed. In many of these cases, the shaped porous body serves as the carrier for the active catalytic species. In filtration applications, complex shaped bodies such as honeycombs are often employed. Here the shaped porous body can either be the discriminating material or, more frequently, the carrier for a discriminating layer, such as in a membrane device as exemplified in Auriol, et al., U.S. Patent No. 4,724,028. The ability to produce a shaped porous body having the desired properties is of value.

Weber et al., U.S. Patent No. 4,379,134 describes high purity alpha-alumina bodies, at least 85 percent of the pore volume of the bodies having pores with a diameter of from 10,000 to 200,000 Angstroms. The high purity alpha-alumina bodies are prepared by peptizing boehmite in an acidic aqueous, fluoride anion-containing mixture. An extrudable mixture is formed thereby which is extruded and shaped into formed bodies which are thereafter dried at 100 to 300°C, calcined at a temperature of from 400 to 700°C, and subsequently calcined further at a temperature of from 1,200 to 1,700°C.

A method of producing granulated porous corundum having- a homogeneous porous structure with a total pore volume of 0.3 to 1.0 cm³/g and a predominant pore size of 5,000 to 30,000 A is described by Kuklina et al., U.S. Patent No. 3,950,507. The method of preparing the alpha-alumina includes treating active alumina or aluminum hydroxide having a porous structure in a first heat treatment in which the temperature is increased from 20 to 700°C, a second heat treatment in the range of from 700 to 1,000°C, and a third treatment in the range of from 1,000 to 1,400°C. Each of the heat treatments is for a period of at least one-half hour, the first heat treatment being conducted in an atmosphere of hydrogen fluoride in which the alumina absorbs the hydrogen fluoride; the second heat treatment desorbs the hydrogen fluoride. The patent also describes a similar procedure employing stationary thermal conditions in which the granules of alumina or aluminum hydroxide are impregnated with other fluorine-containing substances prior to the first thermal treatment.

Notermann, U.S. Patent No. 4,994,589 describes support materials composed of a particulate matrix which includes particles having at least one substantially flat major surface, and lamellate or platelet-type particles which have two, or sometimes more, flat surfaces. Notermann discloses that support materials having flat surfaces may be formed by treating material having another morphology or material lacking the desired flat surface with a suitable agent which serves to recrystallize the material to the desired form. Notermann exemplifies the use of NH₄F to treat gamma alumina pills or rings, followed by heating the treated pills or rings in a high temperature furnace for one hour to raise the temperature to 700°C, four hours to raise the temperature from 700 to 1,000°C, and a hold temperature at 1100°C for one hour.

Mohri, et al., U.S. Patent No. 5,538,709 describes a process for producing alpha-alumina powder comprising alpha-alumina single crystal particles. The process comprises calcining at least one of transition alumina and a transition alumina precursor capable of becoming transition alumina on heating, in a gas atmosphere containing (1) a halogen selected from fluorine, chlorine, bromine, and iodine, (2) a hydrogen halide selected from hydrogen fluoride, hydrogen bromide, and hydrogen iodide, or (3) a component prepared from a halogen gas selected from fluorine gas, bromine gas, and iodine gas, and steam.

Yeates, et al., US 2006/0014971 describes "fluoride-mineralized carriers" obtained by combining alpha-alumina or alpha-alumina precursor(s) with a fluorine-containing species that is capable of liberating fluoride, typically as hydrogen fluoride, when the combination is calcined, and calcining the combination.

Jin, et al., EP 0327356B1 discloses a process for preparing silver containing catalysts and their carriers. The carrier is made from a so-called tri-hydrated alpha-alumina and boehmite mixed with carbonaceous material, a flux, a fluoride, water and a binder. The preferred fluorides are ammonium fluoride, hydrogen fluoride, and aluminum fluoride. The materials are dried and calcined at a temperature of 1450 to 1550°C and kept at that temperature for 2 to 6 hours.

Wallin, et al., US 6,953,554 discloses a mixture of precursor compounds such as aluminum, silicon, and oxygen, heated under an atmosphere sufficient to form a porous catalyst support. Any suitable temperature and atmosphere may be used depending on the chemistry of the ceramic particles of the porous catalyst desired. For example, when forming mullite, at least during some portion of the heating of the precursor compounds, fluorine is present in the atmosphere from sources, such as SiF₄, AlF₃, HF, Na₂SiF₆, NaF, and NH₄F. The porous body, when making mullite, is generally heated to a first temperature for a time sufficient to convert the precursor compounds in the porous body to fluorotopaz and then raised to a second temperature sufficient to form the mullite composition. The temperature may also be cycled between the first and second temperature to ensure complete mullite formation. After mullite formation, the porous body may be treated to reduce the amount of fluoride ions in the article.

U.S. Patent No. 4,994,588 relates to a direct oxidation of alkenes to the corresponding epoxides in which the stability of the catalyst is enhanced by the presence of a fluorine-containing substance.

### SUMMARY OF THE INVENTION

This invention is embedded within a broader disclosure. The invention is as defined in the appended claim set.

The broader disclosure is directed to improved methods of preparing shaped porous bodies comprising platelets of alpha-alumina. In certain embodiments, the methods allow better control of the synthesis conditions than can be achieved with previously known methods. In some embodiments, using the methods of the present invention results in products having properties unachievable by previously known methods. For example, in embodiments of the methods of the invention the surface composition of the alpha-alumina can be modified with a greater degree of control than was possible using the processes of the prior art. In some embodiments, less fluorine is wasted as compared to prior art processes, because the fluorine-containing gas can be recovered and re-used.

In one aspect of the disclosure, not claimed in the present invention, is described herein a method for making a shaped porous body, where the shaped porous body comprises alpha-alumina platelets. The method comprises providing a vessel in which a total pressure can be varied and controlled and introducing a shaped precursor body into the vessel. The shaped precursor body comprises at least one alpha-alumina precursor. The method further comprises introducing into the vessel, as a fluorine-containing gas at the conditions of introduction, a fluorine-containing compound. A total pressure within the vessel of between about 1 torr and 100,000 torr ( 13 3 Pa to 13.3 MPa) is established and the fluorine-containing gas contacts at least a portion of the shaped precursor body. The contacting occurs at one or more temperatures, for one or more pressures, and for one or more time periods, sufficient to convert at least 50% of the alpha-alumina precursor to alpha-alumina platelets.

In another aspect of the disclosure, not claimed in the present invention, is described a method for making a shaped porous body where the shaped porous body comprises alpha-alumina platelets. The method comprises providing a vessel and introducing a shaped precursor body into the vessel. The shaped precursor body comprises at least one alpha-alumina precursor. The method further comprises heating the vessel to a temperature above 700°C, and introducing into the heated vessel, as a fluorine-containing gas at the conditions of introduction, a fluorine-containing compound. The fluorine-containing gas contacts at least a portion of the shaped precursor body, at least one temperature above 700°C, for one or more time periods, sufficient to convert at least 50% of the alpha-alumina precursor to alpha-alumina platelets.

In a third aspect of the disclosure, not claimed as part of the present invention is described a method for making a shaped porous body where the shaped porous body comprises alpha-alumina platelets. The method comprises providing a vessel and introducing a shaped precursor body into the vessel. The shaped precursor body comprises at least one alpha-alumina precursor. The method further comprises introducing into the vessel, as a fluorine-containing gas at the conditions of introduction, a fluorine-containing compound not consisting essentially of hydrogen fluoride. The fluorine-containing gas contacts at least a portion of the shaped precursor body, at one or more temperatures, and for one or more time periods, sufficient to convert at least 50% of the alpha-alumina precursor to alpha-alumina platelets.

The shaped precursor body comprises at least one alpha-alumina precursor. The shaped precursor body may comprise a fluorine-containing compound. The shaped precursor may be free or essentially free of a fluorine-containing compound. The shaped precursor body may comprise alpha-alumina in addition to at least one alpha-alumina precursor. In one embodiment, the shaped precursor body further comprises a modifier.

The fluorine-containing gases which can be used in the methods described but not claimed as part of the present invention may comprise one or more organic or inorganic fluorine-containing compounds, or mixtures of one or more organic and inorganic fluorine-containing compounds. Examples of such organic or inorganic fluorine-containing compounds include halofluorides, flurorocarbons, halofluorocarbons, HF, SiF₄, BF₃, NF₃, F₂, XeF₂, SF₆, PF₅, CF₄, CHF₃, C₂H₂F₄, AlF₃ or mixtures of two or more of the fluorine-containing compounds. The fluorine-containing gas may also comprise, for example, inert gases.

In a vessel in which a total pressure can be varied and controlled, the total pressure ranges from 1 to 100,000 torr (133 Pa to 13.3 MPa).

Except in the embodiments where the temperature is at least 700°C, the temperature at which the gas contacts the shaped precursor body as described herein may range from about 25 to about 1600°C.

In one embodiment of the disclosure, that is not part of the claimed invention, the method described further comprises removing, after the contacting of the fluorine-containing gas with at least a portion of the shaped precursor body, at least a portion of the remaining fluorine-containing gas from the vessel. In one embodiment, this is followed by heating the vessel under vacuum to a second temperature in the range of about 1050 to about 1600°C for between about 1 hour and about 48 hours.

In one embodiment, the method described by the disclosure further comprises placing the vessel under a vacuum prior to introducing the fluorine-containing gas.

In a further embodiment, the method described by the disclosure further comprises removing at least a portion of the remaining fluorine-containing gas and, introducing into the vessel, as a fluorine- containing gas at the conditions of introduction, a second fluorine-containing compound. The same fluorine-containing compounds described above can be used in this embodiment. In another embodiment, the method described by the disclosure further comprises removing at least a portion of the remaining second fluorine-containing gas.

Additional embodiments of the methods described by the disclosure comprise cooling (or allowing to cool) the shaped body comprising alpha-alumina platelets. In one embodiment, the alpha-alumina platelets produced by the methods of the invention have an aspect ratio (as defined, *infra)* of at least about 5.

The invention provides a method for modifying the surface composition of alpha-alumina. The method according to the invention comprises providing a vessel (which is a vessel in which a total pressure can be varied and controlled) and introducing alpha-alumina having a surface composition into the vessel. The method further comprises introducing into the vessel, as a fluorine-containing gas at the conditions of introduction, a fluorine-containing compound selected from the group consisting of SiF₄, BF₃, CF₄, CHF₃, C₂H₂F₄ or mixtures of two or more of the gases HF, SiF₄, BF₃, CF₄, CHF₃ and C₂H₂F₄. The fluorine-containing gas is contacted with at least a portion of the alpha-alumina for one or more periods of time, at a total pressure ranging from 1 to 100,000 torr (133 Pa to 13.3 MPa) and at temperatures between 750 and 1200 °C to modify the surface composition of the alpha-alumina. In one embodiment of the method of the invention, a shaped precursor body comprises the alpha-alumina. In another embodiment of the method of the invention, the alpha-alumina comprises platelets. In a further embodiment of the method of the invention, the shaped body comprises alpha-alumina comprising platelets.

In a vessel in which a total pressure can be varied and controlled, the total pressure ranges from 1 to 100,000 torr (133 Pa to 13.3 MPa).

The same fluorine-containing compounds are selected from the group consisting of SiF₄, BF₃, CF₄, CHF₃, C₂H₂F₄ or mixtures of two or more of the **gases** HF, SiF₄, BF₃, CF₄, CHF₃ and C₂H₂F₄.

In one embodiment of the method of the invention, the alpha-alumina has a purity of at least about 90 percent, exclusive of modifier. The fluorine-containing gas contacts at least a portion of the alpha- alumina. The contact is maintained for such times and at such temperatures and pressures that the surface composition of the alpha-alumina is changed. The temperatures at which the gas may contact the alpha-alumina may range from 750 to 1200°C.

In one embodiment of the method of the invention, the method further comprises placing the vessel under a vacuum prior to introducing the fluorine-containing gas. Additional embodiments of the method of the invention comprise cooling (or allowing to cool) the alpha-alumina having a modified surface composition.

In one embodiment of the method of the invention, the source of the alpha-alumina having the surface composition to be modified is a product of one of the methods of the present invention for producing shaped porous bodies comprising alpha-alumina platelets.

In a further aspect, the disclosure describes a carrier for a catalyst. The catalytic component or components can be added before, during, or after the carrier preparation. Catalysts include, but are not limited to, metals, metal oxides, organic or inorganic components, a combination of ceramic platelet particles having metal deposited on them, an enzyme or enzyme supported by wash coats, a metal active site supported on a high surface area carbon, organometallic molecules, and catalysts for the epoxidation of an olefin. The catalyst may be directly bound to the surface of the carrier platelet particles, incorporated into the lattice structure of the carrier platelet particles, or bound to a washcoat which has been applied to the carrier.

The carriers may comprise shaped porous bodies comprising alpha-alumina produced by one or more of the methods of the present invention. If the alpha-alumina comprises platelets, the aspect ratio of the alpha-alumina platelets preferably is at least about 5.

In additional embodiments (not according to the claimed invention), the shaped porous bodies comprising alpha-alumina platelets and the alpha-alumina having a modified surface composition produced by one or more methods of the present invention can also be used as carriers for the epoxidation of an olefin.

The present disclosure describes the products produced by the foregoing methods, including alpha-alumina having a modified surface composition and shaped porous bodies comprising alpha-alumina platelets.

In another embodiment, the alpha-alumina having a modified surface composition and the shaped porous bodies comprising alpha-alumina platelets can be used to make carriers for catalysts used in a process for the epoxidation of olefins. In one embodiment, the carrier for the epoxidation of an olefin comprises at least about 80 percent by weight alumina, and of that alumina, at least about 90 percent by weight is alpha-alumina, exclusive of modifier, and the surface area is at least about 1.0 m²/g. In one embodiment, the carrier has a porosity of at least 75 percent and an average flat plate crush strength of at least about 1lb/mm (0.45 kg/mm), measured as a hollow cylinder having an axial cylindrical opening the length of the cylinder, an Outer Diameter ("O.D.") of about 0.26 inches (0.66 cm) and an Inner Diameter ("I.D.") of the opening of about 0.1 inches (0.25 cm) and a length of about 0.25 inches (0.64 cm). In one embodiment, the carrier has a porosity of at least about 70 percent and an average flat plate crush strength of at least about 3.5 lb/mm (1.6 kg/mm), measured in the same manner. In one embodiment, the alpha-alumina in the carrier comprises platelets.

### DESCRIPTION OF THE DRAWINGS

Fig. 1 depicts the relationship between porosity and flat plate crush strength for various carriers.
Fig. 2 depicts the results of scanning electron microscopy of a random sample of the platelets produced in Example No. 1.
Fig. 3 depicts the results of scanning electron microscopy of a random sample of the platelets produced in Example No. 2.
Fig. 4 depicts the results of scanning electron microscopy of a random sample of the platelets produced in Example No. 3.
Fig. 5 depicts the results of scanning electron microscopy of a random sample of the platelets produced in Example No.7.
Fig. 6 depicts the results of scanning electron microscopy of a random sample of the platelets produced in Example No. 32.
Fig. 7 depicts the results of scanning electron microscopy of a random sample of the platelets produced in Example No. 36.

### DETAILED DESCRIPTION OF THE INVENTION

### Definitions

The following definitions and methods are provided to better define the present invention and to guide those of ordinary skill in the art in the practice of the present invention. Unless otherwise noted, terms are to be understood according to conventional usage by those of ordinary skill in the relevant art.

An "alpha-alumina precursor" means one or more materials capable of being transformed into alpha-alumina, including transition aluminas as well as materials such as aluminum trihydroxides and aluminum oxide hydroxides.

"Aspect ratio" means the ratio of the longest or major dimension to the smallest or minor dimension of the platelet particles.

"Carrier" and "support" are interchangeable terms. A carrier provides surface(s) to deposit, for example, catalytic metals, metal oxides, or promoters that a component of a catalyst.

"Crush Strength Average and Range" can be determined according to ASTM Method No. D 6175-98.

"Fluorine-containing gas" means a gaseous chemical compound or mixture of compounds at least one of which contains the element fluorine. Fluorine-containing gases include, but are not limited to, HF, SiF₄,BF₃, NF₃, F₂, XeF₂, SF₆, PF₅, halofluorides, CF₄, CHF₃, C₂H₂F₄, AlF₃, fluorocarbons, halofluorocarbons, and mixtures of two or more of such gases.

"Modifier" means a component other than alumina, added to a precursor or shaped porous body to introduce desirable properties such as improved catalyst performance. Non-limiting examples of such modifiers include zirconium silicate, see WO 2005/039757, and alkali metal silicates and alkaline earth metal silicates, see WO 2005/023418 and metal oxides, mixed metal oxides, for example, oxides of cerium, manganese, tin, and rhenium.

"Partial pressure" as used herein means the fraction of the total pressure of a mixture of gases that is due to one component of the mixture.

The term "platelet" means that a particle has at least one substantially flat major surface, and that some of the particles have two, or sometimes more, flat surfaces. The "substantially flat major surface" referred to herein may be characterized by a radius of curvature of at least about twice the length of the major dimension of the surface. The platelets frequently have a morphology which approximates the shape of small plates or wafers. Preferably, a majority of the platelets are "interfused" or "interpenetrated" or "interlocking" platelets, that is, having the appearance of platelets growing out of or passing through one another at various angles. In some cases the edges of some of the platelet or wafer-like particles contact or are fused to the faces of other particles to provide a structure which appears to be an irregular "house of cards" structure. A platelet is a particular shape of a particle. More preferably, at least about 75 percent, still more preferably, at least about 85 percent, and most preferably, at least about 90 percent of the platelets are "interfused" or "interpenetrated" or "interlocking" platelets.

"Total pore volume" is typically determined by mercury porosimetry. The measurements reported herein used the method described in Webb & Orr, Analytical Methods in Fine Particle Technology (1997), p. 155, using mercury intrusion to 60,000 psia (413.7 MPa) using Micrometries Autopore IV 9520, assuming 130° contact angle, 0.473 N/M surface tension of Hg.

"Porosity" is the proportion of the non-solid volume to the total volume of material. Total pore volume as measured by mercury porosimetry or water absorption may be used to estimate porosity by those of skill in the art.

A "shaped precursor body" is defined as a solid which has been formed into a selected shape suitable for its use and in the composition in which it will be contacted with the fluorine-containing gas. Suitable shapes of shaped precursor bodies include, but are not limited to, pills, chunks, tablets, pieces, spheres, pellets, tubes, wagon wheels, toroids having star shaped inner and/or outer surfaces, cylinders, hollow cylinders, amphora, rings, Raschig rings, honeycombs, monoliths, saddles, cross-partitioned hollow cylinders (e.g. having at least one partition extending between walls), cylinders having gas channels from side wall to side wall, cylinders having two or more gas channels, and ribbed or finned structures. While the cylinders are often circular, other cross-sections, such as oval, hexagonal, quadrilateral, trilateral may be useful.

As used herein, "shaped porous body" means a solid with porosity greater than about 10 percent which has been formed into a selected shape suitable for its use. Suitable shapes of porous bodies for use as catalyst carriers in fixed bed reactors include, but are not limited to, pills, chunks, tablets, pieces, spheres, pellets, tubes, wagon wheels having star shaped inner and/or outer surfaces, cylinders, hollow cylinders, amphora, Raschig rings, honeycombs, monoliths, saddles, cross-partitioned hollow cylinders (e.g. having at least one partition extending between walls), cylinders having gas channels from side wall to side wall, cylinders having two or more gas channels, and ribbed or finned structures. While the cylinders are often circular, other cross-sections, such as oval, hexagonal, quadrilateral, trilateral may be useful. The shaped porous bodies can be of sizes and shapes suitable for employment in fixed bed reactors or fluidized bed reactors.

"Surface area" as used herein refers to the surface area as determined by the BET (Brunauer, Emmett and Teller) method by nitrogen as described in the Journal of the American Chemical Society 60 (1938) pp. 309-316.

"Surface composition" is the chemical composition of the first one to 50 atomic layers of a solid material. Surface composition is commonly measured by XPS. Other methods such as Auger electron spectroscopy ("AES"), ion scattering spectroscopy ("ISS") may also be employed but may have a different sampling depth thus measuring a concentration different than those used here.

"Transition aluminas" are one or more aluminas other than alpha-alumina, which are capable of being at least partially converted to alpha-alumina under thermal treatment at 1100°C or greater. Transition aluminas possess varying degrees of crystallinity, and include, but are not limited to gamma-alumina, delta-alumina, eta-alumina, kappa-alumina, chi- alumina, rho-alumina, and theta-alumina.

"Transition alumina precursors" are one or more materials that upon thermal treatment are capable of being at least partially converted to transition alumina. Transition alumina precursors include, but are not limited to, aluminum tri-hydroxide, such as gibbsite, bayerite, and nordstrandite, aluminum oxide hydroxide, such as boehmite, pseudo-boehmite and diaspore.

"Water absorption" is expressed as the weight of the water than can be absorbed into the pores of the carrier, relative to the weight of the carrier, as measured in accordance with ASTM C393.

### Product Uses/Applications

The products produced by the process of the present invention can be used, for example, as catalyst carriers for reactions including but not limited to epoxidation of alkenes, partial oxidation of methanol to formaldehyde, partial selective oxidation of saturated hydrocarbons to olefins, selective hydroformylation of olefins, selective hydrogenations, selective hydrogenation of acetylenes in cracked hydrocarbon streams, selective hydrogenation of di-olefins in olefin-di-olefin-aromatic streams also known as pyrolysis gasoline, and selective reduction of NOₓ to N₂. The products produced by the process of the present invention may be used other than as catalytic supports. For example, the products produced by the process of the present invention can be used for filtration of materials from liquid or gas streams, see, e.g. Auriol, et al., U.S. Patent No. 4,724,078. In these applications the porous shaped bodies may do the filtration or may be the support for what does the filtration. Other uses include applications as automotive exhaust catalysts for emissions control, packings for distillations and catalytic distillations, and carrier for enzymatic catalysis.

### Methods of Making Shaped Porous Bodies Comprising Alpha-Alumina Platelets

In one aspect, described in the present disclosure is a method for making a shaped porous body, where the shaped porous body comprises alpha-alumina platelets. The method comprises providing a vessel in which a total pressure can be varied and controlled and introducing a shaped precursor body into the vessel. The shaped precursor body comprises at least one alpha- alumina precursor. The method further comprises introducing into the vessel, as a fluorine-containing gas at the conditions of introduction, a fluorine-containing compound. A total pressure within the vessel of between about 1 torr (133 Pa) and 100,000 torr (13.3 MPa) is established and the fluorine-containing gas contacts at least a portion of the shaped precursor body. The contacting occurs at one or more temperatures, for one or more pressures, and for one or more time periods, sufficient to convert at least 50% of the alpha-alumina precursor to alpha-alumina platelets.

In another aspect described in the disclosure, described is a method for making a shaped porous body where the shaped porous body comprises alpha-alumina platelets. The method comprises providing a vessel and introducing a shaped precursor body into the vessel. The shaped precursor body comprises at least one alpha-alumina precursor. The method further comprises heating the vessel (which may, but does not have to be a vessel in which a total pressure can be varied and controlled) to a temperature above 700°C, and introducing into the heated vessel, as a fluorine-containing gas at the conditions of introduction, a fluorine- containing compound. The fluorine-containing gas contacts at least a portion of the shaped precursor body, at least one temperature above 700°C, for one or more time periods, sufficient to convert at least 50 % of the alpha-alumina precursor to alpha-alumina platelets.

In a third aspect, described in this disclosure is a method for making a shaped porous body where the shaped porous body comprises alpha-alumina platelets. The method comprises providing a vessel (which may, but does not have to be a vessel in which a total pressure can be varied and controlled) and introducing a shaped precursor body into the vessel. The shaped precursor body comprises at least one alpha-alumina precursor. The method further comprises introducing into the vessel, as a fluorine-containing gas at the conditions of introduction, a fluorine-containing compound not consisting essentially of hydrogen fluoride. The fluorine-containing gas contacts at least a portion of the shaped precursor body, at one or more temperatures, and for one or more time periods, sufficient to convert at least 50 % of the alpha-alumina precursor to alpha-alumina platelets.

The vessels used in the **disclosure and** in the methods of the present invention require the ability to heat the samples to elevated temperature. Many such vessels, commonly called furnaces, are known. Examples of commonly used furnaces are box kilns and rotary kilns. Unless specifically modified, these furnaces are vessels in which a total pressure cannot be varied and controlled. Examples of suitable vessels in which a total pressure can be varied and controlled include controlled atmosphere furnaces commonly used for the sintering of non- oxide ceramics and/or treatment of metals, as exemplified by the controlled atmosphere furnaces made by Centorr Vacuum Industries (Nashua NH, USA), and the quartz reaction vessel described in Wallin, et al., U.S. Patent No. 6,306,335. The shaped precursor body comprises at least one alpha-alumina precursor. Preferably the shaped precursor body comprises at least 75 percent by weight, more preferably 80 percent by weight, even more 85 percent by weight and most preferably 90 percent by weight alpha-alumina precursor. Depending on the application for which the shaped porous body comprising alpha-alumina platelets will be used, the preferred amount of alpha-alumina precursor will differ. The shaped precursor body may comprise a fluorine-containing compound. The shaped precursor may be free or essentially free of a fluorine-containing compound. The shaped precursor body may comprise alpha-alumina in addition to at least one alpha alumina precursor.

The fluorine-containing compound is introduced as a fluorine-containing gas at the conditions of introduction. Thus, fluorine-containing compounds which are not in gas phase at atmospheric pressure and room temperature may be converted to a fluorine- containing gas by methods known to those skilled in the art. The fluorine-containing gases which can be used in the methods of the present disclosure are selected from the group consisting of organic and inorganic fluorine-containing compounds, and mixtures of organic and inorganic fluorine-containing compounds. Except in the embodiments where the fluorine-containing gas does not consist essentially of HF: (1) such organic or inorganic fluorine-containing compounds are selected from the group consisting of halofluorides, fluorocarbons, halofluorocarbons, HF, SiF₄, BF₃, NF₃, F₂, XeF₂, SF₆, PF₅, CF₄, CHF₃, C₂H₂F₄,and AlF₃ and mixtures of two or more of these gases; (2) preferred fluorine- containing gases are selected from the group consisting of HF, SiF₄, BF₃, CF₄, CHF₃, C₂H₂F₄, and mixtures of two or more of these gases; (3) in one embodiment, the fluorine- containing gas is selected from the group consisting of HF, SiF₄, C₂H₂F₄,and BF₃, and mixtures of two or more of these gases; (4) in one embodiment, the fluorine-containing gas is HF; (5) in one embodiment, the fluorine-containing gas is SiF₄; (6) in one embodiment, the fluorine-containing gas is C₂H₂F₄; and (7) in one embodiment, the fluorine-containing gas is BF₃.

In a vessel in which a total pressure can be varied and controlled, the total pressure ranges from about 1 to about 100,000 torr (133 Pa to 13.3 MPa), preferably from about 1 to about 2000 torr ( 133 Pa to about 266.6 kPa) and more preferably from about 10 to about 760 torr (1.3 kPa to about 101.3 kPa).

The contact is maintained for such time and at such temperatures and/or pressures that at least about 50 percent, 55 percent, 60 percent, 65 percent, 70 percent, 75 percent, 80 percent, 85 percent or more of the alpha-alumina precursor is converted to alpha-alumina platelets. Preferably, at least about 90 percent, more preferably, at least about 95 percent, and most preferably at least about 99 percent of the alpha-alumina precursor is converted to alpha-alumina platelets as measured by x-ray diffraction. Except in those embodiments where the temperature is at least 700°C, the temperatures at which the gas may contact the alpha-alumina may range from about 25 to about 1600°C. The temperature may be 50, 200, 300, 400, 500, 600, 700, 800, 900, 1000, 1100, 1200, 1300, 1500, or 1500°C or temperatures in between. Preferably, the temperature range is from about 400°C to about 1200°C more preferably from about 750 to about 1200°C, and most preferably, from about 850 to about 1100°C. The gas may contact the shaped precursor body at a temperature in the ranges given. The temperature range of contact is broader than the temperature range at which the alpha-alumina precursor is converted to alpha-alumina platelets.

Without wishing to be bound by theory, it is believed that there are, in general, three temperatures at which the gas may initially contact the shaped precursor body comprised of alpha-alumina precursor. The first is a temperature at which no reaction between the gas and the precursor occurs or no reaction appreciably occurs. This temperature is a low temperature, generally below 600°C. The second is a temperature at which a reaction occurs between the gas and the precursor, but this reaction does not result in the conversion of the precursor to alpha-alumina platelets. For example, the reaction of transition alumina with SiF₄ at temperatures of 680 to 800° C, will cause AlF₃ and fluorotopaz to form, but not alpha-alumina platelets. (Further heating to higher temperature will result in the formation of alpha-alumina platelets.). The third is a temperature at which the reaction between the gas and the precursor results in the conversion of the precursor to alpha-alumina platelets. For example, reaction of transition alumina with SiF₄ at temperatures of 900 to 1000°C results in the formation of alumina platelets. The disclosure is intended to encompass contacting the fluorine-containing gas and the shaped precursor body at any initial temperature, whether or not the initial temperature of contact between the fluorine- containing gas and the transition alumina is sufficient to cause a reaction.

The methods disclosed comprise heating the vessel containing the shaped precursor body to a temperature in a range of about 750 to about 1150°C prior to introducing the fluorine-containing gas, the fluorine-containing gas establishes a partial pressure of between about 1 torr and about 10,000 torr (133 Pa to 1.33 MPa), and the shaped precursor body is contacted with the fluorine-containing gas for a time of about 1 minute to about 48 hours. The partial pressure may be 1, 100, 200, 300, 400, 500, 600, 700, 800, 900, 1000, 2500, 5000, 7500, or 10,000 torr (133 Pa, 13,3 kPa, 26.7 kPa, 40 kPa, 53.3 kPa, 66.7 kPa, 80 kPa, 93.3 kPa, 106.7 kPa, 120 kPa, 133.3 kPa, 333.3 kPa, 666.6 kPa, 999.9 kPa, 1333.2 kPa) or pressures in between. A preferred partial pressure is below 760 torr (101.3 kPa). The time may be 1 minute, 15 minutes, 30 minutes, 45 minutes, 1 hour, 90 minutes, 2 hours, 3 hours, 4 hours, 5 hours, 10 hours, 20 hours, 30 hours, 40 hours or about 48 hours or times in between. Shorter times for contacting the gas with the shaped precursor body are preferred.

In one embodiment, the temperature is in a range of about 850 to about 1050°C. In another embodiment, the temperature is in a range of about 900 to about 1000°C. In one embodiment, the time is about 30 to about 90 minutes. In one embodiment, the vessel is heated to a first temperature in the range of about 850 to about 1150°C prior to introducing the fluorine-containing gas and then heated to a second temperature greater than the first temperature and between about 950 and about 1150°C after introducing the fluorine-containing gas. In another embodiment, the first temperature is increased to the second temperature at a rate of about 0.2 to about 4°C per minute.

The preferred combinations of time and temperature and/or pressure vary with the fluorine-containing gas used. For SiF₄, a preferred time and temperature combination is between about 30 minutes and about 6 hours at a temperature of between about 900 to about 1000°C. A preferred pressure, time, and temperature combination is about 300 torr (40 kPa), about 1 to about 2 hours, at about 950°C. Using BF₃ the preferred combination may be a temperature of about 880 to about 950°C, a pressure of about 50 to about 300 torr (6.7 kPa to about 40 kPa), for a time between about 15 min and about 2 hrs. For HFC-134a, a preferred process is to load the precursor into the reactor and heat under a vacuum to a temperature between about 750 and 850°C. The gas is then added to the reactor until a pressure of about 25 to 300 torr (3.3 kPa to 40 kPa) is achieved. The precursor bodies are allowed to contact the gas for about one to four hours at the initial temperature and then the reactor is heated to a temperature of above at least 900°C.

In another embodiment, the temperature at which the fluorine-containing gas is introduced to the vessel containing the shaped precursor body is insufficient to convert the alpha-alumina precursor to alpha-alumina platelets. The gas-contacted, heated shaped precursor body is then heated to a second temperature for a time sufficient for the fluorine-containing gas to convert at least 50% of the alpha-alumina precursor to alpha-alumina platelets.

In one embodiment, the method further comprises removing, after the contacting of the fluorine-containing gas with at least a portion of the shaped precursor body, at least a portion of the remaining fluorine-containing gas from the vessel followed by heating the vessel under vacuum to a second temperature in the range of about 1050 to about 1600°C for between about 1 hour and about 48 hours.

In one embodiment, the method further comprises placing the vessel under a vacuum prior to introducing the fluorine-containing gas.

In a further embodiment, the method further comprises removing at least a portion of the remaining fluorine-containing gas and, introducing into the vessel, as a fluorine-containing gas at the conditions of introduction, a second fluorine-containing compound. The fluorine-containing gases which can be used in the methods disclosed herein are selected from the group consisting of organic and inorganic fluorine-containing compounds, and mixtures of organic and inorganic fluorine-containing compounds. The fluorine- containing gases are selected from the group consisting of halofluorides, fluorocarbons, halofluorocarbons, HF, SiF₄, BF₃, NF₃, F₂, XeF₂, SF₆, PF₅, CF₄, CHF₃, C₂H₂F₄, and AlF₃ and mixtures of two or more of these gases. Preferred fluorine-containing gases are selected from the group consisting of HF, SiF₄, BF₃, CF₄, CHF₃, C₂H₂F₄, and mixtures of two or more of these gases. In one embodiment, the fluorine-containing gas is selected from the group consisting of HF, C₂H₂F₄, SiF₄, and BF₃, and mixtures of two or more of these gases. In one embodiment, the fluorine-containing gas is HF. In one embodiment, the fluorine- containing gas is SiF₄. In one embodiment, the fluorine-containing gas is BF₃. In one embodiment, the fluorine-containing gas is C₂H₂F₄. In another embodiment, the method further comprises removing at least a portion of the remaining second fluorine-containing gas.

Additional embodiments of the methods described comprise cooling (or allowing to cool) the shaped body comprising alpha-alumina platelets. In one embodiment, the alpha-alumina platelets produced by the methods described have an aspect ratio of at least about 5.

Shaped porous bodies comprising at least one alpha-alumina precursor can be prepared by mixing precursor compounds, shaping the mixture, and optionally thermally treating the shaped mixture. Generally, the mixture of precursor compounds is comprised of hydrated aluminum compounds, such as boehmite, gibbsite, or bayerite, or transition aluminas obtained by thermal dehydration of the hydrated aluminum compounds. For carriers used for the epoxidation of alkylene, the preferred alpha-alumina precursors comprise pseudoboehmite, gibbsite, gamma-alumina and kappa-alumina.

The mixture of precursor compounds may also contain precursor catalyst compounds that have elements that may be incorporated onto the surface or into the lattice structure of the alpha-alumina particles that will be formed upon contact with the fluorine containing gas. Examples of compounds useful for forming these incorporated catalysts include inorganic and organic compounds that form catalysts such as metals, metal oxides, metal carbides and organo-metallic compounds.

Other organic compounds may also be used to facilitate the shaping of the mixture (e.g., binders and dispersants, such as those described in Introduction to the Principles of Ceramic Processing, J. Reed, Wiley Interscience, 1988) or to alter the porosity of the mixture. Pore formers (also known as burn out agents) are materials used to form specially sized pores in the shaped porous body by being burned out, sublimed, or volatilized. Pore formers are generally organic, but examples of inorganic pore formers are known. The pore formers are usually added to the precursor raw materials prior to shaping. During a drying or calcining step or during the conversion of the alpha-alumina precursor to alpha-alumina, the pore formers are burned out, sublimed, or volatilized. The use of natural and synthetic organic components, such as ground walnut shells, granulated polyolefins, such as polyethylene and polypropylene which are later heated to high enough temperature to incinerate and leave voids in the shaped body made from the mixture is well known in the art and may also be used with the presently described methods.

The mixture may be made by any suitable method, such as those known in the art. Examples include ball milling, mix-mulling, ribbon blending, vertical screw mixing, V-blending, and attrition milling. The mixture may be prepared dry (i.e., in the absence of a liquid medium) or wet.

The mixture is then shaped into a porous shape by any suitable method, such as those known in the art. Examples include injection molding, extrusion, isostatic pressing, slip casting, roll compaction and tape casting. Each of these is described in more detail in Introduction to the Principles of Ceramic Processing, J. Reed, Chapters 20 and 21, Wiley Interscience, 1988.

Modifiers may be added to the precursor raw materials or the shaped precursor bodies to change the chemical and/or physical properties of the shaped porous body or products made from such shaped bodies. The modifier can be added at one or more steps in the process. For example, a metal oxide modifier can be added to the precursor raw materials. A modifier can be added to the shaped precursor body before or during the conversion to a shaped porous body. Alternatively, a modifier can be added to a shaped porous body. Modifiers can be inorganic compounds or naturally occurring minerals which are added in order to impart properties such as strength and, in some cases, change the surface chemical properties of the carrier and/or catalyst. In one embodiment of the methods disclosed, the greater degree of control of the conversion to alpha-alumina in the process described can be used to incorporate the components of the modifier into the carrier. (By contrast, a binder is used in the precursor material to hold the raw materials together and have the proper consistency for extrusion.) Non-limiting examples of a modifier are silicon-containing modifier, such as alumino-silicates, alkali metal silicates, alkaline earth metal silicates, alkali metal alumino-silicates alkaline earth metal alumino-silicates, transition metal silicates, transition metal alumino-silicates, metal oxides, mixed metal oxides, for example, oxides of cerium, manganese, tin, and rhenium, feldspars and clays.

The mixture of precursor compounds may be heated under an atmosphere sufficient to remove water, decompose any organic additives, or otherwise modify the shaped precursor body prior to introduction into the reaction vessel for contact with a fluorine-containing gas. Suitable atmospheres include, but are not limited to air, nitrogen, argon, hydrogen, carbon dioxide, water vapor, or any combination of mixtures thereof.

### Methods for Modifying the Surface Composition of Alpha-Alumina

The process of the invention may be used to purposely alter the surface composition of the shaped porous body comprising alpha-alumina platelets, either during the conversion of alpha-alumina precursor to alpha-alumina or after such conversion. To measure the surface composition of the alpha-alumina platelets, i.e. the elemental composition of the first one to 50 atomic layers of the platelets, an analytical technique such as XPS may be used. The surface composition can be altered by the addition of new elements or by the depletion of elements which are impurities in the raw materials used to produce the shaped precursor bodies. The time, temperature and/or pressure of the process of the invention may be varied and controlled in such way as to cause the surface composition of the platelets to contain elements other than aluminum, fluorine and oxygen. As examples, the platelet composition may be altered to include silicon-containing species by the use of SiF₄ or to include boron-containing species by the use of BF₃ as the fluorine-containing gas of the process. These modifications are in addition to modifications introduced by modifiers added to the shaped precursor body.

In another aspect, the invention is a method for modifying the surface composition of alpha-alumina. The method comprises providing a vessel and introducing alpha-alumina having a surface composition into the vessel. The method further comprises introducing into the vessel, as a fluorine-containing gas at the conditions of introduction, a fluorine-containing compound. The fluorine-containing gas is contacted with at least a portion of the alpha-alumina for one or more periods of time, and at one or more temperatures sufficient to modify the surface composition of the alpha-alumina. In one embodiment, a shaped body comprises the alpha-alumina. In another embodiment, the alpha-alumina comprises platelets. In a further embodiment, the shaped body comprises alpha-alumina comprising platelets.

The vessel used in this embodiment is be a vessel in which a total pressure can be varied and controlled.

The fluorine-containing gases which can be used in the methods of the present invention are selected from the group consisting of organic and inorganic fluorine- containing compounds, and mixtures of organic and inorganic fluorine-containing compounds. The fluorine-containing gases are selected from the group consisting of SiF4, BF3, CF₄, CHF₃, C₂H₂F₄ and mixtures of two or more of HF, SiF₄, BF₃, CF₄, CHF₃, C₂H₂F₄. In one embodiment, the fluorine-containing gas is selected from the group consisting of SiF₄, C₂H₂F₄, and BF₃, and mixtures of two or more of HF, SiF₄, C₂H₂F₄, and BF₃. In one embodiment, the fluorine-containing gas is HF. In one embodiment, the fluorine-containing gas is SiF_{4.} In one embodiment, the fluorine-containing gas is BF₃. In one embodiment, the fluorine-containing gas is C₂H₂F₄.

In a vessel in which a total pressure can be varied and controlled, the total pressure ranges from 1 to 100,000 torr (133 Pa to 13.3 MPa). The contact is maintained for such time and at such temperatures and pressures that the surface composition of the alpha-alumina is changed. The temperatures at which the gas may contact the alpha-alumina may range from about 25 to about 1600°C. The temperature range is from 750 to 1200°C, and most preferably, from about 750 to about 1100°C.

In one embodiment, the method further comprises placing the vessel under a vacuum prior to introducing the fluorine-containing gas. Additional embodiments of the methods comprise cooling (or allowing to cool) the alpha-alumina having a modified surface composition.

In one embodiment, the alpha-alumina is in the form of a shaped body. In one embodiment, the alpha-alumina comprises platelets. In one embodiment, the alpha-alumina platelets are in the form of a shaped body.

In one embodiment, the source of the alpha-alumina having the surface composition to be modified is a product of one of the methods of the present invention for producing shaped porous bodies comprising alpha-alumina platelets.

The modification of the surface composition can be measured by XPS.

### Increasing Crush Strength

In one aspect described by this disclosure, there is a method of increasing the crush strength of the shaped porous bodies comprising alpha-alumina platelets. The method comprises heat treatment of the bodies in an air atmosphere subsequent to platelet formation. The heat treatment may take place in the reactor after platelet formation or in a separate heat treatment unit. The temperature of the heat treatment is greater than about 1000°C. Preferably, the temperature is greater than about 1100°C, more preferably greater than about 1200°C, still more preferably greater than about 1300°C, and most preferably greater than about 1400°C.

### Alpha-alumina Platelets

In an additional aspect described by this disclosure, described is an alpha-alumina platelet comprising a surface composition comprising silicon-containing species with a concentration of about 1 to about 20 atom percent silicon as measured by XPS. In addition to the silicon, the first one to 50 atomic layers of the platelet may comprise fluorine or boron or mixture of silicon, fluorine and boron. In yet another aspect, the alpha-alumina platelet comprises a surface composition comprising boron-containing species with a concentration of about 1 to about 20 atom percent boron as measured by XPS. In addition to the boron, the first one to 50 atomic layers of the platelet may comprise fluorine or silicon or a mixture of boron, fluorine, and silicon.

### Carriers for Catalysts

In another aspect, the **disclosure describes** a carrier for a catalyst. The catalytic species can be bound directly on the surface of the platelet particles comprising the product of the invention. The catalytic species may also be bound to another surface which has been applied to the surface of the platelet particles comprising the product of the invention. This applied surface is commonly known as a washcoat. Washcoats include one or more inorganic oxides and carbon. The catalytic species may also be covalently attached to a macromolecular species, such as synthetic polymer or a biopolymer such as a protein or nucleic acid polymers, which in tum is bound either directly to the surface of the platelet particles comprising the product of the invention or to an applied washcoat. The catalytic species may reside on the surface of the alpha-alumina platelets, be incorporated into the lattice of the alpha-alumina platelets, or be in the form of discrete particles interspersed among the alpha-alumina platelets. The catalytic species may also comprise the product of the invention. For example, the porous shaped precursor body may comprise an appropriate metal oxide component to give the product of the invention a desired catalytic activity.

A first preferred type of catalytic species are metal catalysts, such as noble metals, base metals and combinations thereof Examples of noble metal catalysts include gold, platinum, rhodium, palladium, ruthenium, rhenium, silver and mixtures thereof. Examples of base metal catalysts include copper, chromium, iron, cobalt, nickel, zinc, manganese, vanadium, titanium, scandium and combinations thereof. The metal may be applied by any suitable technique, such as those known in the art. For example, the metal catalyst may be applied by solution impregnation, physical vapor deposition, chemical vapor deposition or other techniques.

A second type of preferred catalyst species is a solid state compound such as an oxide, nitride and carbide. An example is a perovskite-type catalyst comprising a metal oxide composition, such as those described by Golden, U.S. Patent No. 5,939,354.

A third type of preferred catalytic species is a molecular catalyst such as a metal Schiff base complex, a metal phosphine complex or a diazaphosphacycle. These catalytic species can be covalently attached directly to the ceramic particles of the carrier, to a wash coat such as silica, alumina or carbon, or to a supported high surface area carbon such as carbon nanofibers. Immobilization methods include those generally known to those skilled in the art such as attachment through silane coupling agents.

A fourth preferred catalytic species is an enzyme or enzyme supported by wash coats or high surface area carbon. The enzyme may also be supported by other suitable supports such as those known in the art. Preferred supports for the enzyme include carbon nanofibers such as those described by Kreutzer, WO2005/084805A1, polyethyenimine, alginate gels, sol-gel coatings, or combinations thereof. Preferably, the enzyme is a lipase, a lactase, a dehalogenase or combinations thereof. More preferably the enzyme is a lipase, a lactase or combination thereof.

The amount of catalyst may be any suitable amount depending on the particular use. Generally, at least about 10 percent to essentially all of the ceramic particles are coated or contain a catalyst.

In one embodiment of the first preferred type of catalytic species a preferred type of carrier is a carrier for a catalyst for the epoxidation of an olefin. The carrier comprises at least about 80 percent by weight alumina, and of that alumina, at least about 90 percent by weight is alpha-alumina, exclusive of modifier, and the surface area is at least about 1.0 m²/g. In one embodiment, the carrier has a porosity of at least 75 percent and an average flat plate crush strength of at least about 1 lb/mm (0.45 kg/mm), measured as a hollow cylinder having an axial cylindrical opening the length of the cylinder, an O.D. of about 0.26 inches (0.66 cm) and an I.D. of the opening of about 0.1 inches (0.25 cm) and a length of about 0.25 inches (0.64 cm). In one embodiment, the carrier has a porosity of at least about 70% and an average flat plate crush strength of at least about 3.5 lb/mm (1.6 kg/mm), measured in the same manner. In one embodiment, the alpha-alumina in the carrier comprises platelets.

In additional embodiments, the shaped porous bodies comprising alpha-alumina platelets and the alpha-alumina having a modified surface composition produced by one or more methods of the present invention can also be used as carriers. Those of skill in the art will appreciate that not all modified surface compositions will be appropriate for all types of catalysts and that appropriate selection or optimization may be required.

### Products Produced by the Methods/Using the Compositions

The present disclosure describes the products produced by the foregoing methods or using the foregoing compositions, including alpha- alumina having a modified surface composition and the shaped porous bodies comprising alpha-alumina platelets.

### Catalysts for Epoxidation of Olefins

The following discussion is presented in terms of and with reference to ethylene oxide made by the epoxidation of ethylene for the sake of simplicity and illustration. However, the scope and range of the present disclosure is generally applicable to catalysts for the epoxidation of suitable alkenes and mixtures thereof.

The production of alkylene oxide, such as ethylene oxide, by the reaction of oxygen or oxygen-containing gases with ethylene in the presence of a silver-containing catalyst at elevated temperature is described in Liu, et al., U.S. Patent No. 6,511,938 and Bhasin, U.S. Patent No. 5,057,481. See also Kirk- Othmer's Encyclopedia of Chemical Technology, 4th Ed. (1994) Volume 9, pages 915 to 959.

The catalyst is an important factor in direct oxidation of ethylene to produce ethylene oxide. There are several well-known basic components of such catalyst: the active catalyst metal (generally silver as described above); a suitable support/carrier; and catalyst promoters, all of which can play a role in improving catalyst performance.

### The Carriers

In commercially useful catalysts for the production of ethylene oxide, the carrier upon which the silver and promoters reside must have a physical form and strength to allow proper flow of gaseous reactants, products and ballast gas(es) through the reactor while maintaining physical integrity over catalyst life. Significant catalyst breakage or abrasion is highly undesirable because of the pressure drop and safety problems such degradation can cause. The catalyst must also be able to withstand fairly large temperature fluctuations within the reactor. The pore structure and chemical inertness of the carrier are also important factors that must be considered for optimum catalyst performance.

In general, the carriers are made up of an inert, refractory support, such as alpha-alumina, having a porous structure and relatively high surface area. Generally, for catalysts for use in the epoxidation of alkenes, improved results have been demonstrated when the support material is compositionally pure and also phase pure. By "compositionally pure" is meant a material which is substantially a single substance, such as alumina, with only trace impurities being present. "Phase purity" or like terms refer to the homogeneity of the support with respect to its phase. In the present invention, alumina, having a high or exclusive alpha-phase purity (i.e., alpha-alumina), is preferred. Most preferred is a material composed of at least 98 percent, by weight, of alpha-alumina exclusive of modifier.

In some catalyst applications a high purity alpha-alumina carrier is highly desirable. For these applications, a precursor consisting essentially of an alpha- alumina precursor is preferred. One method of obtaining such a precursor is to extrude a mixture comprising a alpha-alumina precursor (e.g. pseudo-boehmite or gibbsite), an organic binder (e.g. methylcellulose), an organic lubricant (e.g. polyethylene glycol) and, optionally, an organic pore former (e.g. nut shell flour, polypropylene or polyethylene fibers or powders) followed by cutting, drying and debindering/calcining in air.

In other epoxidation catalyst applications, a primarily alpha-alumina carrier having minor silicate and/or other oxide components containing alkaline earth metal, transition metal, rare earth or main group elements is highly desirable, particularly when these minor oxide components are in combination with silicon. Such compositions can readily be achieved by the process of this invention either by adding the minor components as pure oxides or salts, or if desired as mixed oxides or salts, to the precursor before shaping or by adding the minor components via either solution or gas phase infiltration after shaping. Common additives for formation of minor phases giving improved catalyst performance include silicates, alumino-silicates, borates, alkaline earth metal containing compounds, transition metal element-containing compounds, rare earth element-containing compounds, and main group element-containing compounds.

As with other carriers, modifiers may be added to the precursor raw materials, shaped precursor body, or shaped porous body.

Suitable shapes for the alpha-alumina carrier include any of the wide variety of shapes known for such carriers or supports, including, but not limited to, pills, chunks, tablets, pieces, spheres, pellets, tubes, wagon wheels, toroids having star shaped inner and/or outer surfaces, cylinders, hollow cylinders, amphora, rings, Raschig rings, honeycombs, monoliths, saddles, cross-partitioned hollow cylinders (e.g. having at least one partition extending between walls), cylinders having gas channels from side wall to side wall, cylinders having two or more gas channels, and ribbed or finned structures. While the cylinders are often circular, other cross-sections, such as oval, hexagonal, quadrilateral, trilateral may be useful. The size of the shapes should be suitable for employment in fixed bed reactors.

Conventional commercial fixed bed ethylene oxide reactors are typically in the form of a plurality of parallel elongated tubes (in a suitable shell) about 1 to 3 inches (2.5 to 7.5 cm) outer diameter and about 15 to 45 feet (4.5 to 13.5 m) long filled with catalyst. In such fixed bed reactors, it is desirable to employ a carrier formed into a rounded shape, such as, for example, spheres, pellets, rings, tablets, and the like, having diameters from about 0.1 inch (0.25 cm) to about 0.8 inch (2 cm).

The alpha-alumina carrier preferably has a specific surface area of at least about 0.5 m²/g, and more preferably, at least about 0.7 m²/g. The surface area is typically less than about 10 m²/g, and preferably, less than about 5m²/g. The high-purity alumina carrier preferably has a total pore volume of at least about 0.5 mL/g, and more preferably, from about 0.5 mL/g to about 2.0 mL/g; and a median pore diameter from about 1 to about 50 microns. The high-purity alpha-alumina preferably includes particles which have at least one substantially flat major surface having a lamellate or platelet morphology which approximates the shape of a hexagonal plate (some particles having two or more flat surfaces), at least 50 percent of which (by number) have a major dimension of less than about 50 microns.

The carriers may comprise shaped porous bodies comprising alpha-alumina produced by one or more of the methods of the present invention. The alpha-alumina is preferably at least about 90 percent alpha-alumina platelets, more preferably at least about 95 percent alpha-alumina platelets, and even more preferably at least about 99 percent alpha-alumina platelets. The aspect ratio of the alpha-alumina platelets preferably is at least about 5.

Catalysts for the production of alkylene oxide, for example, ethylene oxide or propylene oxide may be prepared with the aforementioned carriers by impregnating the carrier with a solution of one or more silver compounds, depositing the silver throughout the pores of the carrier and reducing the silver compound as is well known in the art. See for example, Liu, et al., U.S. Patent No. 6, 511,938 and Thorsteinson et al., U.S. Patent No. 5,187,140.

Generally, the carrier is impregnated with a catalytic amount of silver, which is any amount of silver capable of catalyzing the direct oxidation of the alkylene with oxygen or an oxygen-containing gas to the corresponding alkylene oxide. In making such a catalyst, the carrier is typically impregnated (one or more times) with one or more silver compound solutions sufficient to allow the silver to be supported on the carrier in an amount greater than about 5 percent, greater than about 10 percent, greater than about 15 percent, greater than about 20 percent, greater than about 25 percent, preferably, greater than about 27 percent, and more preferably, greater than about 30 percent by weight, based on the weight of the catalyst. Typically, the amount of silver supported on the carrier is less than about 70 percent, and more preferably, less than about 50 percent by weight, based on the weight of the catalyst.

Although silver particle size in the finished catalyst is important, the range is not narrow. A suitable silver particle size can be in the range of from about 10 to about 10,000 angstroms in diameter. A preferred silver particle size ranges from greater than about 100 to less than about 5,000 angstroms in diameter. It is desirable that the silver be relatively uniformly dispersed within, throughout, and/or on the alumina carrier.

As is known to those skilled in the art, there are a variety of known promoters, that is, materials which, when present in combination with particular catalytic materials, for example, silver, benefit one or more aspect of catalyst performance or otherwise act to promote the catalyst's ability to make a desired product, for example ethylene oxide or propylene oxide. Such promoters in themselves are generally not considered catalytic materials. The presence of such promoters in the catalyst has been shown to contribute to one or more beneficial effects on the catalyst performance, for example enhancing the rate or amount of production of desired product, reducing the temperature required to achieve a suitable rate of reaction, reducing the rates or amounts of undesired reactions, etc. Competing reactions occur simultaneously in the reactor, and a critical factor in determining the effectiveness of the overall process is the measure of control one has over these competing reactions. A material which is termed a promoter of a desired reaction can be an inhibitor of another reaction, for example a combustion reaction. What is significant is that the effect of the promoter on the overall reaction is favorable to the efficient production of the desired product, for example ethylene oxide. The concentration of the one or more promoters present in the catalyst may vary over a wide range depending on the desired effect on catalyst performance, the other components of a particular catalyst, the physical and chemical characteristics of the carrier, and the epoxidation reaction conditions.

There are at least two types of promoters--solid promoters and gaseous promoters. The solid and/or gaseous promoters are provided in a promoting amount. A "promoting amount" of a certain component of a catalyst refers to an amount of that component that works effectively to provide an improvement in one or more of the catalytic properties of that catalyst when compared to a catalyst not containing said component.

Examples of well-known solid promoters for catalysts used to produce ethylene oxide include compounds of potassium, rubidium, cesium, rhenium, sulfur, manganese, molybdenum, and tungsten. During the reaction to make ethylene oxide, the specific form of the promoter on the catalyst may be unknown. Examples of solid promoter compositions and their characteristics as well as methods for incorporating the promoters as part of the catalyst are described in Thorsteinson et al., U.S. Patent No. 5,187,140, particularly at columns 11 through 15, Liu, et al., U.S. Patent 6,511,938, Chou et al., U.S. Patent No. 5,504,053, Soo, et al., U.S. Patent No. 5,102, 848, Bhasin, et al., U.S. Patent Nos. 4, 916,243, 4,908,343, and 5,059,481, and Lauritzen, U.S. Patent Nos. 4,761,394, 4,766,105, 4,808,738, 4,820,675, and 4,833,261. The solid promoters are generally added as chemical compounds to the catalyst prior to its use. As used herein, the term "compound" refers to the combination of a particular element with one or more different elements by surface and/or chemical bonding, such as ionic and/or covalent and/or coordinate bonding. The term "ionic" or "ion" refers to an electrically charged chemical moiety; "cationic" or "cation" being positive and "anionic" or "anion" being negative. The term "oxyanionic" or "oxyanion" refers to a negatively charged moiety containing at least one oxygen atom in combination with another element. An oxyanion is thus an oxygen-containing anion. It is understood that ions do not exist in vacuo, but are found in combination with charge- balancing counter ions when added as a compound to the catalyst. Once in the catalyst, the form of the promoter is not always known, and the promoter may be present without the counterion added during the preparation of the catalyst. For example, a catalyst made with cesium hydroxide may be analyzed to contain cesium but not hydroxide in the finished catalyst. Likewise, compounds such as alkali metal oxide, for example cesium oxide, or transition metal oxides, for example MoO₃, while not being ionic, may convert to ionic compounds during catalyst preparation or in use. For the sake of ease of understanding, the solid promoters will be referred to in terms of cations and anions regardless of their form in the catalyst under reaction conditions.

The catalyst prepared on the carrier may contain alkali metal and/or alkaline earth metal as cation promoters. Exemplary of the alkali metal and/or alkaline earth metals are lithium, sodium, potassium, rubidium, cesium, beryllium, magnesium, calcium, strontium and barium. Other cation promoters include Group 3b metal ions including lanthanide series metals. In some instances, the promoter comprises a mixture of cations, for example cesium and at least one other alkali metal, to obtain a synergistic efficiency enhancement as described in U.S. No. 4,916,243. Note that references to the Periodic Table herein shall be to that as published by the Chemical Rubber Company, Cleveland, Ohio, in CRC Handbook of Chemistry and Physics, 46th Edition, inside back cover.

The concentration of the alkali metal promoters in the finished catalyst is not narrow and may vary over a wide range. The optimum alkali metal promoter concentration for a particular catalyst will be dependent upon performance characteristics, such as catalyst efficiency, rate of catalyst aging and reaction temperature.

The concentration of alkali metal (based on the weight of cation, for example cesium) in the finished catalyst may vary from about 0.0005 to 1.0 wt. %, preferably from about 0.005 to 0.5 wt. %. The preferred amount of cation promoter deposited on or present on the surface of the carrier or catalyst generally lies between about 10 and about 4000, preferably about 15 and about 3000, and more preferably between about 20 and about 2500 ppm by weight of cation calculated on the total carrier material. Amounts between about 50 and about 2000 ppm are frequently most preferable. When the alkali metal cesium is used in mixture with other cations, the ratio of cesium to any other alkali metal and alkaline earth metal salt(s), if used, to achieve desired performance is not narrow and may vary over a wide range. The ratio of cesium to the other cation promoters may vary from about 0.0001:1 to 10,000:1, preferably from about 0.001:1 to 1,000: 1. Preferably, cesium comprises at least about 10, more preferably, about 20 to 100, percent (weight) of the total added alkali metal and alkaline earth metal in finished catalysts using cesium as a promoter.

Examples of some of the anion promoters which may be employed with the present disclosure include the halides, for example fluorides and chlorides, and the oxyanions of the elements other than oxygen having an atomic number of 5 to 83 of Groups 3b to 7b and 3a to 7a of the Periodic Table. One or more of the oxyanions of nitrogen, sulfur, manganese, tantalum, molybdenum, tungsten and rhenium may be preferred for some applications.

The types of anion promoters or modifiers suitable for use in the catalysts of this disclosure comprise, by way of example only, oxyanions such as sulfate, SO₄⁻², phosphates, for example, PO₄⁻³, titanates, e.g., TiO₃⁻² , tantalates, for example, Ta₂O₆⁻², molybdates, for example, MoO₄⁻², vanadates, for example, V₂O₄ ⁻², chromates, for example, CrO₄⁻², zirconates, for example, ZrO₃⁻² polyphosphates, manganates, nitrates, chlorates, bromates, borates, silicates, carbonates, tungstates, thiosulfates, cerates and the like. The halides may also be present, including fluoride, chloride, bromide and iodide.

It is well recognized that many anions have complex chemistries and may exist in one or more forms, for example, orthovanadate and metavanadate; and the various molybdate oxyanions such as MoO₄ ⁻², and Mo₇O₂₄⁻⁶ and Mo₂O₇⁻². The oxyanions may also include mixed metal-containing oxyanions including polyoxyanion structures. For instance, manganese and molybdenum can form a mixed metal oxyanion. Similarly, other metals, whether provided in anionic, cationic, elemental or covalent form may enter into anionic structures.

While an oxyanion, or a precursor to an oxyanion, may be used in solutions impregnating a carrier, it is possible that during the conditions of preparation of the catalyst and/or during use, the particular oxyanion or precursor initially present may be converted to another form. Indeed, the element may be converted to a cationic or covalent form. In many instances, analytical techniques may not be sufficient to precisely identify the species present.

When the promoter comprises rhenium, the rhenium component can be provided in various forms, for example, as the metal, as a covalent compound, as a cation or as an anion. The rhenium species that provides the enhanced efficiency and/or activity is not certain and may be the component added or that generated either during preparation of the catalyst or during use as a catalyst. Examples of rhenium compounds include the rhenium salts such as rhenium halides, the rhenium oxyhalides, the rhenates, the perrhenates, the oxides and the acids of rhenium. However, the alkali metal perrhenates, ammonium perrhenate, alkaline earth metal perrhenates, silver perrhenates, other perrhenates and rhenium heptoxide can also be suitably utilized. Rhenium heptoxide, Re₂O₇, when dissolved in water, hydrolyzes to perrhenic acid, HReO₄, or hydrogen perrhenate. Thus, for purposes of this specification, rhenium heptoxide can be considered to be a perrhenate, that is, ReO₄. Similar chemistries can be exhibited by other metals such as molybdenum and tungsten.

Another class of promoters, which may be employed with the present disclosure, includes manganese components. In many instances, manganese components can enhance the activity, efficiency and/or stability of catalysts. The manganese species that provides the enhanced activity, efficiency and/or stability is not certain and may be the component added or that generated either during catalyst preparation or during use as a catalyst. Manganese components include, but are not limited to, manganese acetate, manganese ammonium sulfate, manganese citrate, manganese dithionate, manganese oxalate, manganous nitrate, manganous sulfate, and manganate anion, for example permanganate anion, and the like. To stabilize the manganese component in certain impregnating solutions, it may be necessary to add a chelating compound such as ethylenediaminetetraacetic acid (EDTA) or a suitable salt thereof.

The amount of anion promoter may vary widely, for example, from about 0.0005 to 2 wt. %, preferably from about 0.001 to 0.5 wt. % based on the total weight of the catalyst. When used, the rhenium component is often provided in an amount of at least about 1, say, at least about 5, for example, about 10 to 2000, often between 20 and 1000, ppmw calculated as the weight of rhenium based on the total weight of the catalyst.

The promoters for catalyst may also be of the type comprising at least one efficiency-enhancing salt of a member of a redox-half reaction pair which is employed in an epoxidation process in the presence of a gaseous nitrogen-containing component capable of forming a gaseous efficiency-enhancing member of a redox-half reaction pair under reaction conditions. The term "redox-half reaction" is defined herein to mean half-reactions like those found in equations presented in tables of standard reduction or oxidation potentials, also known as standard or single electrode potentials, of the type found in, for instance, "Handbook of Chemistry", N. A. Lange, Editor, McGraw-Hill Book Company, Inc., pages 1213-1218 (1961) or "CRC Handbook of Chemistry and Physics", 65th Edition, CRC Press, Inc., Boca Raton, Fla., pages D155-162 (1984). The term "redox-half reaction pair" refers to the pairs of atoms, molecules or ions or mixtures thereof which undergo oxidation or reduction in such half-reaction equations. Such terms as redox-half reaction pairs are used herein to include those members of the class of substance which provide the desired performance enhancement, rather than a mechanism of the chemistry occurring. Preferably, such compounds, when associated with the catalyst as salts of members of a half reaction pair, are salts in which the anions are oxyanions, preferably an oxyanion of a polyvalent atom; that is, the atom of the anion to which oxygen is bonded is capable of existing, when bonded to a dissimilar atom, in different valence states. As used herein, the term "salt" does not indicate that the anion and cation components of the salt be associated or bonded in the solid catalyst, but only that both components be present in some form in the catalyst under reaction conditions. Potassium is the preferred cation, although sodium, rubidium and cesium may also be operable, and the preferred anions are nitrate, nitrite and other anions capable of undergoing displacement or other chemical reaction and forming nitrate anions under epoxidation conditions. Preferred salts include KNO₃ and KNO₂, with KNO₃ being most preferred.

The salt of a member of a redox-half reaction pair is added to the catalyst in an amount sufficient to enhance the efficiency of the epoxidation reaction. The precise amount will vary depending upon such variables as the gaseous efficiency-enhancing member of a redox-half reaction used and concentration thereof, the concentration of other components in the gas phase, the amount of silver contained in the catalyst, the surface area of the support, the process conditions, for example space velocity and temperature, and morphology of support. Alternatively, a suitable precursor compound may also be added such that the desired amount of the salt of a member of a redox-half reaction pair is formed in the catalyst under epoxidation conditions, especially through reaction with one or more of the gas-phase reaction components. Generally, however, a suitable range of concentration of the added efficiency-enhancing salt, or precursor thereof, calculated as cation, is about 0.01 to about 5%, preferably about 0.02 to about 3%, by weight, based on the total weight of the catalyst. Most preferably the salt is added in an amount of about 0.03 to about 2 wt. %.

The preferred gaseous efficiency-enhancing members of redox-half reaction pairs are compounds containing an element capable of existing in more than two valence states, preferably nitrogen and another element which is, preferably, oxygen. The gaseous component capable of producing a member of a redox-half reaction pair under reaction conditions is a generally a nitrogen-containing gas, such as for example nitric oxide, nitrogen dioxide and/or dinitrogen tetroxide, hydrazine, hydroxylamine or ammonia, nitroparaffins (for example, nitromethane), nitroaromatic compounds (for example nitrobenzene), N-nitro compounds, and nitriles (for example, acetonitrile). The amount of nitrogen-containing gaseous promoter to be used in these catalysts is that amount sufficient to enhance the performance, such as the activity of the catalyst and particularly the efficiency of the catalyst. The concentration of the nitrogen- containing gaseous promoter is determined by the particular efficiency-enhancing salt of a member of a redox-half reaction pair used and the concentration thereof, the particular alkene undergoing oxidation, and by other factors including the amount of carbon dioxide in the inlet reaction gases. For example, U.S. Patent 5504053 discloses that when the nitrogen-containing gaseous promoter is NO (nitric oxide), a suitable concentration is from about 0.1 to about 100 ppm, by volume, of the gas stream.

Although in some cases it is preferred to employ members of the same half-reaction pair in the reaction system, that is, both the efficiency-enhancing salt promoter associated with the catalyst and the gaseous promoter member in the feedstream, as, for example, with a preferred combination of potassium nitrate and nitric oxide, this is not necessary in all cases to achieve satisfactory results. Other combinations, such as KNO₂/N₂O₃, KNO₃/NO₂, KNO_{3/}N₂O₄, KNO₂/NO, KNO₂/NO₂ may also be employed in the same system. In some instances, the salt and gaseous members may be found in different half-reactions which represent the first and last reactions in a series of half-reaction equations of an overall reaction.

In any event, the solid and/or gaseous promoters are provided in a promoting amount. Examples of catalytic properties include, inter alia, operability (resistance to run-away), selectivity, activity, conversion, stability and yield. It is understood by one skilled in the art that one or more of the individual catalytic properties may be enhanced by the "promoting amount" while other catalytic properties may or may not be enhanced or may even be diminished. It is further understood that different catalytic properties may be enhanced at different operating conditions. For example, a catalyst having enhanced selectivity at one set of operating conditions may be operated at a different set of conditions wherein the improvement shows up in the activity rather than the selectivity and an operator of an ethylene oxide plant will intentionally change the operating conditions in order to take advantage of certain catalytic properties even at the expense of other catalytic properties in order to maximize profits by taking into account feedstock costs, energy costs, by-product removal costs and the like.

The promoting effect provided by the promoters can be affected by a number of variables such as for example, reaction conditions, catalyst preparative techniques, surface area and pore structure and surface chemical properties of the support, the silver and copromoter content of the catalyst, the presence of other cations and anions present on the catalyst. The presence of other activators, stabilizers, promoters, enhancers or other catalyst improvers can also affect the promoting effects.

It is desirable that the silver and one or more solid promoters be relatively uniformly dispersed on the carrier. A preferred procedure for depositing silver catalytic material and one or more promoters comprises: (1) impregnating a carrier with a solution comprising a solvent or solubilizing agent, silver complex and one or more promoters, and (2) thereafter treating the impregnated carrier to convert the silver compound and effect deposition of silver and the promoter (s) onto the exterior and interior pore surfaces of the carrier. Silver and promoter depositions are generally accomplished by heating the solution containing carrier at elevated temperatures to evaporate the liquid within the carrier and effect deposition of the silver and promoters onto the interior and exterior carrier surfaces. Impregnation of the carrier is the preferred technique for silver deposition because it utilizes silver more efficiently than coating procedures, the latter being generally unable to effect substantial silver deposition onto the interior surfaces of the carrier. In addition, coated catalysts are more susceptible to silver loss by mechanical abrasion.

The gaseous promoters are gas-phase compounds and or mixtures thereof which are introduced to a reactor for the production of alkylene oxide (for example ethylene oxide) with vapor-phase reactants, such as ethylene and oxygen. Such promoters, also called modifiers, inhibitors or enhancers, further enhance the performance of a given catalyst, working in conjunction with or in addition to the solid promoters. One or more chlorine- containing components are typically employed as gaseous promoters, as is well known in the art. See, for example, Law, et al., U.S. Patent Nos. 2,279,469 and 2,279,470. Other halide-containing components may also be used to produce a similar effect.

Depending on the composition of the solid catalyst being employed, one or more gaseous components capable of generating at least one efficiency-enhancing member of a redox half reaction pair may be employed as gaseous promoters, as is well known in the art. The preferred gaseous component capable of generating an efficiency-enhancing member of a redox half reaction pair is preferably a nitrogen-containing component. See, for example, Liu, et al., U.S. Patent No. 6,511,938 particularly at column 16, lines 48 through 67 and column 17, line 28, and Notermann, U.S. Patent No. 4,994,589, particularly at column 17, lines 10-44. As used herein, the term "salt" does not indicate that the anion and cation components of the salt be associated or bonded in the solid catalyst, but only that both components be present in some form in the catalyst under reaction conditions.

Alternatively, a suitable precursor compound may also be added such that the desired amount of the salt of a member of a redox-half reaction pair is formed in the catalyst under epoxidation conditions, especially through reaction with one or more of the gas-phase reaction components. The suitable range of concentration of the precursor of the efficiency enhancing promoter is the same as for the salt.

Well known methods can be employed to analyze for the amounts of silver and solid promoters deposited onto the alumina carrier. The skilled artisan may employ, for example, material balances to determine the amounts of any of these deposited components. Alternatively, any suitable analytical technique for determining elemental composition, such as X-ray fluorescence (XRF), may be employed to determine the amounts of the deposited components.

The present disclosure is applicable to epoxidation reactions in any suitable reactor, for example, fixed bed reactors, continuous stirred tank reactors (CSTR), and fluid bed reactors, a wide variety of which are well known to those skilled in the art and need not be described in detail herein. The desirability of recycling unreacted feed, or employing a single-pass system, or using successive reactions to increase ethylene conversion by employing reactors in series arrangement can also be readily determined by those skilled in the art. The particular mode of operation selected is usually dictated by process economics. Conversion of olefin (alkylene), preferably ethylene, to olefin oxide, preferably ethylene oxide, can be carried out, for example, by continuously introducing a feed stream containing alkylene (e.g., ethylene) and oxygen or an oxygen-containing gas to a catalyst-containing reactor at a temperature of from about 200 to about 300°C, and a pressure which may vary within the range of from about 5 atmospheres (506 kPa) to about 30 atmospheres (3.0 MPa), depending upon the mass velocity and productivity desired. Residence times in large-scale reactors are generally on the order of about 0.1 to about 5 seconds. Oxygen may be supplied to the reaction in an oxygen-containing stream, such as, air or as commercial oxygen, or as oxygen-enriched air. The resulting alkylene oxide, preferably, ethylene oxide, is separated and recovered from the reaction products using conventional methods.

The alkylene oxide produced using the catalyst of the present disclosure or by the method of the present disclosure may be converted into alkylene glycols, alkanolamines and glycol ethers. Ethylene glycol is used in two significant applications: as a raw material for poly(ethylene terephthalate) for use in polyester fiber, film, and containers, and as an automotive antifreeze. Di-, tri-, and tetraethylene glycols are coproducts of ethylene glycol. Ethylene glycol can be produced by the (catalyzed or uncatalyzed) hydrolysis of ethylene oxide. Ethylene oxide hydrolysis proceeds with either acid or base catalysis or uncatalyzed in neutral medium. Acid-catalyzed hydrolysis activates the ethylene oxide by protonation for the reaction with water. Base-catalyzed hydrolysis results in considerably lower selectivity to ethylene glycol. A principal by-product is diethylene glyco and higher glycols, triethylene and tetraethylene glycols, are also produced. Ethylene glycol monoethers can be manufactured by reaction of an alcohol with ethylene oxide. Ethanolamine can be manufactured by the reaction of ethylene oxide with ammonia. See, e.g., US 4845296.

The following examples are set forth for the purpose of illustrating the invention; but these examples are not intended to limit the invention in any manner. One skilled in the art will recognize a variety of substitutions and modifications of the examples that will fall within the scope of the invention.

### EXAMPLES

In the following examples, the supplier of each product used is identified only the first time the product appears in any example. The supplier for that product remains the same in the subsequent examples, unless otherwise identified.

Unless otherwise noted, the controlled atmosphere furnace reactor system referred to in the examples consists of a 6" (15.2 cm) I.D. x 19" (48.3 cm) tall quartz tube closed at one end, the closed end inserted into a furnace so that the top about 5" (12.7cm) protrudes out of the furnace. A reactor chamber is sealed to a stainless steel lid with a Viton gasket. The lid is insulated with Ni disks which serve as radiation shields. The lid is fitted with ports to allow for gas addition and removal and for the insertion of thermocouples into the reactor. A gas manifold allows for the control of the addition and removal of gas from the reactor.

Unless otherwise specified, the XPS measurements referred to in the following examples were conducted as follows: The XPS measurements were performed on a Physical Electronics Model 5600 Multi-technique Surface Analysis System. The analyses were performed using a monochromator anode aluminum X-ray source [Kα=1486.6eV (eV is electron volt)] at 345 watts (15KeV and 23 mA current). The signal was acquired from 800 micron by 800 micron analysis area for general surface characterization. XPS surveys were acquired at 187.5eV pass energy. The C is peak at 284.8eV was used as binding energy (Eb) charge reference. The sample was loaded as received in a pellet form (pellet size about 9 mm in length and 9 mm in diameter) the pellet was loaded into a shallow hole inside the sample holder. Then, the sample was inserted inside the ultrahigh vacuum chamber (UHV) (pressure inside the UHV chamber is approximately or about 10-¹⁰ torr (1.3⁻⁸ Pa)). The XPS survey of the elements was obtained from a depth of 60 angstrom from the pellet outer surface as received. "Crush Strength Average and Range" can be determined according to ASTM Method No. D 6175-98. The crush strength measurements described in the examples below are conducted by modifying ASTM Method No. D 6175-98 by using a sample of 10 test specimens, and by not drying the specimens prior to testing.

### Example Nos. 1 and 2

For Example Nos. 1 and 2, precursor bodies having the shape of about 1/4" (0,64 cm) O.D. (3/32" (0.24 cm)I.D.) 1/4" (0,64 cm) long rings are prepared as follows. An extrudable paste is prepared by mixing 1 kg ofVersal V-250, a pseudo-boehmite, (UOP LLC, DesPlaines, IL USA), 65g of Methocel^{®} A4M (Dow Chemical Company, Midland MI USA), 850 g of water, 20g of oleic acid (VWR Scientific Products, West Chester PA USA) with a mortar and pestle and then extruded with an 18 mm counter rotating twin-screw extruder equipped with a chopping mechanism at the die outlet. The resulting ring-shaped extrudate pellets are dried for 36 to 72 h at 60°C in flowing air, then slowly heated to 1000°C and held for 2 h at 1000°C to remove the organic components and to dehydrate the Versal V-250.

In Example No. 1, the precursor bodies are loaded into the reactor, and heated under a vacuum to 950°C. SiF₄ gas is then added to the reactor until a pressure of 300 torr (40 kPa) is achieved, the reactor is then closed and the precursor bodies allowed to contact the SiF₄ gas (Voltaix, Somerville NJ USA) for 90 min at 950°C. The gas is then removed from the reactor and the bodies cool to room temperature. SEM analysis of randomly chosen rings show the product is primarily interlocked platelets. See Fig, 2. The surface composition of randomly chosen pellets is determined by XPS and the results are given in Table 1.

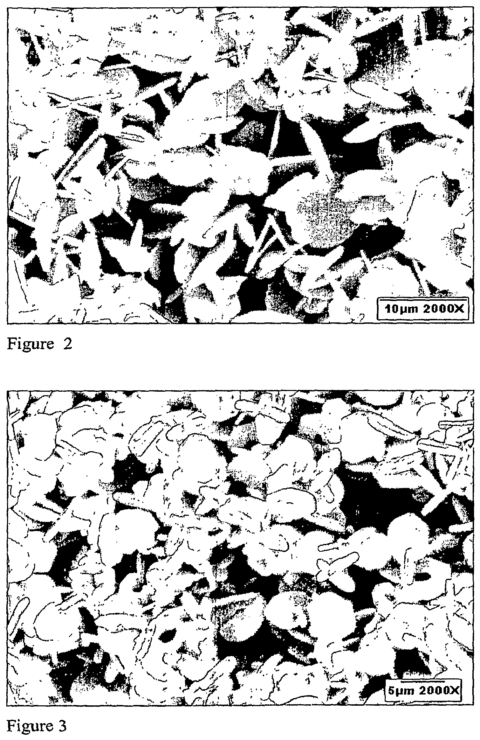

In Example No. 2, the precursor bodies are loaded into the reactor, and heated under a vacuum to 950°C. BF₃ gas (Voltaix, Somerville NJ USA) is then added to the reactor until a pressure of 100 torr (13.3 k P a) is achieved, the reactor is then closed and the precursor bodies allowed to contact the BF₃ gas for 20 min at 950°C. The gas is then removed from the reactor and the bodies cool to room temperature. SEM analysis of randomly chosen rings show the product is primarily interlocked platelets. See Fig. 3. The surface composition of randomly chosen pellets is determined by XPS and the results are given in Table 1.

### Example Nos. 3 through 7

For Example Nos. 3 through 7, precursor bodies having the shape of about ¼" (0.64 cm) O.D. (3/32" (0.24 cm) I.D.) ¼" (0,64 cm) long rings are prepared as follows. An extrudable paste is prepared by mix- mulling 1kg of Versal V-250, a pseudo-boehmite, (UOP LLC, DesPlaines, IL USA), 65g of Methocel^{®} A4M (Dow Chemical Company, Midland MI USA), 850g of water, 20g of oleic acid (VWR Scientific Products, West Chester PA USA) in a stainless steel muller for 12 min. The paste is aged or 24h in a sealed container, and then extruded with an 18 mm counter rotating twin-screw extruder equipped with a chopping mechanism at the die outlet. The resulting ring-shaped extrudate pellets are dried for 36 to 72 h at 60°C in flowing air, then slowly heated to 1000°C and held for 2 h at 1000°C to remove the organic components and to dehydrate the Versal V-250.

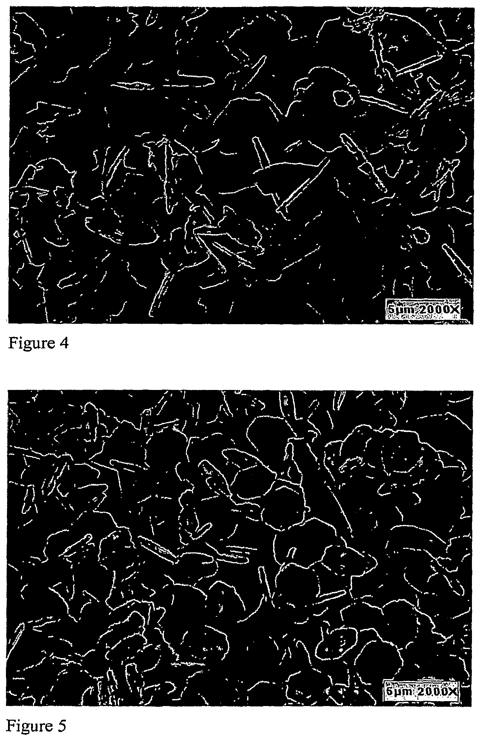

In Example No.3, the precursor bodies are loaded into the reactor, and heated under a vacuum to 950°C. SiF₄ gas is then added to the reactor until a pressure of 300 torr (40 k P a) is achieved, the reactor is then closed and the precursor bodies are allowed to contact the gas for 90 min at 950°C. The gas is then removed from the reactor. Next, BF₃ gas is added to the reactor until a pressure of 180 torr (24 kPa) is achieved, the reactor then closed and the contents allowed to contact the BF₃ gas for 30 min at 950°C. The gas is then removed from the reactor and the bodies cool to room temperature. SEM analysis of randomly chosen rings show the product is primarily interlocked platelets. See Fig. 4. The surface composition of randomly chosen pellets is determined by XPS and the results are given in Table 1.

**Table 1:**

| | Atomic% | | | | | | |
|---|---|---|---|---|---|---|---|
| Example | O | C | Al | Si | F | Na | B |
| 1 | 58.5 | 3.6 | 27.6 | 4.6 | 4.4 | 0.6 | nd |
| 2 | 53.7 | 2.7 | 26.9 | 1.8 | 8.9 | 1.1 | 3.1 |
| 3 | 60.4 | 1.7 | 28.9 | 3.0 | 3.1 | 0.2 | 2.2 |

| | | | | | | | |
|---|---|---|---|---|---|---|---|
| nd = not detected | | | | | | | |

In Example No. 4, the precursor bodies are loaded into the reactor, and heated under a vacuum to 925°C. SiF₄ gas is then added to the reactor until a pressure of 80 torr (10.7 k P a) is achieved; the reactor is then closed and the precursor bodies allowed to contact the SiF₄ gas for 60 min at 925°C. The gas is then removed from the reactor and the bodies cool to room temperature. SEM analysis of randomly chosen rings show the product is primarily interlocked platelets.

In Example No.5, the precursor bodies are loaded into the reactor, and heated under a vacuum to 980°C. SiF₄ gas is then added to the reactor until a pressure of 300 torr (40 k P a) is achieved, the reactor is then closed and the shaped precursor bodies allowed to contact the SiF₄ gas for 90 min at 980°C. The gas is then removed from the reactor and the bodies cool to room temperature. SEM analysis of randomly chosen rings show the product is primarily interlocked platelets.

In Example No.6, the precursor bodies are loaded into the reactor and heated under a vacuum to 950°C. Anhydrous HF gas is added to the reactor until a pressure of 300 torr (40 k P a) is achieved, the reactor closed and the precursor bodies allowed to contact the HF gas for 90 min at 950°C. The gas is then removed from the reactor and the reactor and its contents cool to room temperature.

In Example No.7, the precursor bodies are loaded into the reactor and heated under a vacuum to 950°C. 1,1,1,2-tetrafluoroethane (HFC-134a) (Airgas, Inc., Radnor, Pennsylvania, USA) gas is added to the reactor until a pressure of 300 torr (40 kPa) is achieved, the reactor closed and the precursor bodies allowed to contact the HFC-134a gas for between 5 min and 90 min at 950°C. The gas is then removed from the reactor and the reactor and its contents cool to room temperature. SEM analysis of the randomly chosen rings show the product is primarily interlocked platelets. Fig. 5 depicts the SEM analysis of a randomly chosen ring from contacting the precursor bodies with the gas for 5 minutes at 950°C.

### Example Nos. 8 through 18

For Example Nos. 8 through 18, 100 parts of a precursor to a transition alumina (referred to as the alumina source in Table 2) is mix-mulled with between 2 and 8 parts of a methylcellulose binder (Methocel^{®} A15LV), between 10 and 30 parts of a glycol lubricant (propylene glycol, VWR Scientific Products, West Chester, PA, USA) and between 50 to 80 parts water so as to form an extrudable paste. The paste is extruded with a twin screw extruder into 1/8" (0.32 cm) diameter strands. The precursor is a pseudo-boehmite, Catapal B (UOP LLC, DesPlaines IL USA), Sasol (UOP LLC, DesPlaines IL USA) or Versal V-250 or a gibbsite, Alphabond 300 (Alcoa, Pittsburgh PA, USA) or a mixture of such aluminas (see Table 2). After drying, the extrudates are calcined in air for 2 hours at the temperature given in Table 2. The calcined extrudates are then loaded into a reactor as described in Wallin, et al., U.S. Patent No. 6,306,335 and heated under vacuum to the temperature given in Table 2. The reactor is then filled to the pressure given in Table 2 with SiF₄ gas and the gas is allowed to contact the extrudates for the time given in Table 2. The gas is then removed from the reactor and the reactor cools to room temperature. Samples of the product are examined by SEM microscopy which shows that the product is primarily in the form of interlocked platelets. X-ray diffraction ("XRD") of representative samples show the product is primarily alpha-alumina. The surface area of the product from each Example is determined by N₂ BET and is given in Table 2. The median pore diameter of the product of each Example is determined by mercury porosimetry and is given in Table 2. The pore volume of the product of each Example is determined by mercury porosimetry and is given in Table 2A, as is the pore size distribution.

**Table 2:**

| Example | Alumina Source | Calcination Temp (°C) | SiF₄ Contact Temp (°C) | SiF₄ Pressure (torr) * | Contact Time (min) | BET Surface Area (m²/g) | Median Pore Dia. (µm) |
|---|---|---|---|---|---|---|---|
| 8 | Catapal B | 750 | 925 | 80 | 90 | 1.75 | 2.22 |
| 9 | Catapal B | 750 | 915 | 400 | 90 | 1.74 | 1.81 |
| 10 | Versal V-250 | 750 | 925 | 80 | 90 | 2.29 | 2.28 |
| 11 | Versal V-250 | 750 | 915 | 400 | 90 | 2.27 | 2.26 |
| 12 | Alphabond 300 | 950 | 925 | 80 | 90 | 1.63 | 2.33 |
| 13 | Alphabond 300 | 950 | 915 | 400 | 90 | 1.42 | 2.44 |
| 14 | Alphabond 300 | 750 | 915, held for 20 minutes, then heated to 930 in 20 min, and held for 30 min at 930 | 150 | 70 | 2.60 | 0.779 |
| 15 | 80%Alphabond 20% Catapal B | 950 | 925 | 80 | 90 | 1.65 | 2.32 |
| 16 | 50%Alphabond 50% Catapal B | 750 | 915, held for 20 minutes, then heated to 930 in 20 min and held for 30 min at 930 | 150 | 70 | 2.15 | 1.15 |
| 17 | 20%Alphabond 80% Catapal B | 750 | 925 | 80 | 90 | 1.29 | 2.70 |
| 18 | 20%Alphabond 80% Catapal B | 750 | 915 | 400 | 90 | 1.56 | 2.07 |

| | | | | | | | |
|---|---|---|---|---|---|---|---|
| *80 torr = 10.7 kPa; 150 torr = 20 kPa; 400 torr = 53.3 kPa. | | | | | | | |

**Table 2A:**

| Sample ID | TPV, mL/g (0.003-400 µm pore diameter) | TPV, mL/g (0.003-1 µm pore diameter) | TPV, mL/g (1-5 µm pore diameter) | TPV, mL/g (5-10 µm Pore diameter | TPV, mL/g (10-400 µm pore diameter) |
|---|---|---|---|---|---|
| Example 8 | 0.6742 | 0.0721 | 0.5718 | 0.0092 | 0.0212 |
| Example 9 | 0.6729 | 0.1419 | 0.5069 | 0.0048 | 0.0193 |
| Example 10 | 0.9738 | 0.0631 | 0.8822 | 0.0048 | 0.0237 |
| Example 11 | 0.9818 | 0.0844 | 0.8634 | 0.0064 | 0.0276 |
| Example 12 | 0.9194 | 0.0432 | 0.8269 | 0.0151 | 0.0342 |
| Example 13 | 0.9550 | 0.0299 | 0.8594 | 0.0194 | 0.0463 |
| Example 14 | 0.8674 | 0.6478 | 0.1959 | 0.0043 | 0.0194 |
| Example 15 | 0.9739 | 0.0526 | 0.8428 | 0.0474 | 0.0311 |
| Example 16 | 0.9114 | 0.3756 | 0.5149 | 0.0055 | 0.0155 |
| Example 17 | 0.7628 | 0.0529 | 0.6855 | 0.0037 | 0.0206 |
| Example 18 | 0.7318 | 0.0901 | 0.6138 | 0.0041 | 0.0238 |

| | | | | | |
|---|---|---|---|---|---|
| TPV=Total Pore Volume | | | | | |

### Example Nos. 19 through 22

For Example Nos. 19 and 20, precursor bodies having the shape of 1/4" (0.64 cm) O.D. (3/32" (0.24 cm) I.D.) ¼" (0.64 cm) long rings are prepared as follows. An extrudable paste is prepared by mix- mulling 1kg of Versal V-250, 65g of Methocel A4M, 850g of water, and 20g of oleic acid in a stainless steel muller for 12 min. The paste is then extruded with an 18 mm counter rotating twin-screw extruder equipped with a chopping mechanism at the die outlet. The resulting ring-shaped extrudate pellets are dried for 36 to 72 hours at 60°C in flowing air. For Example No. 19, the dried extrudates are slowly heated to 750°C and held for 2 hours at 750°C to remove the organic components and to dehydrate the Versal V-250. For Example No. 20, the dried extrudates are slowly heated to 950°C and held for 2 hours at 950°C to remove the organic components and to dehydrate the Versal V-250 to form the transition alumina precursor.

For Example Nos. 21 and 22, precursor bodies having the shape of 1/4" (0.64 cm) O.D. (3/32" (0.24 cm) ID.) 1/4" (0.64 cm) long rings are prepared as follows. An extrudable paste is prepared by mix- mulling 1kg of Versal V-250, 65g of Methocel A4M, 850g of water, 20g of oleic acid, and 250g of paraffin wax flakes, (m.p. 65-75°C) (Aldrich, St. Louis, MO USA) in a stainless steel muller for 12 min. The paste is then extruded with an 18 mm counter rotating twin- screw extruder equipped with a chopping mechanism at the die outlet. The resulting ring- shaped extrudate pellets are dried for 36 to 72 hours at 60°C in flowing air. For Example No. 21, the dried extrudates are slowly heated to 750°C and held for 2 hours at 750°C to remove the organic components and to dehydrate the Versal V-250 to form the transition alumina precursor. For Example No. 22, the dried extrudates are slowly heated to 950°C and held for 2 hours at 950°C to remove the organic components and to dehydrate the Versal V-250 to form the transition alumina precursor.

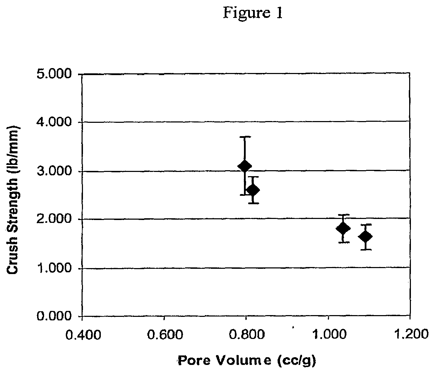

Approximately 45g of each of the respective shaped precursor bodies are loaded into the reactor and heated under a vacuum to 935°C. SiF₄ gas is then added to the reactor until a pressure of 300 torr (40 k P a) is achieved, the reactor is then closed and the transition alumina precursor bodies allowed to contact the SiF₄ gas for 120 min at 935°C. The gas is then removed from the reactor and the bodies cool to room temperature. SEM analysis of randomly chosen rings show the product is primarily interlocked platelets. Pore volume is determined by Hg porosimetry of randomly selected pellets. The flat plate crush strength of 10 randomly selected pellets from each Example is measured and the average flat plate crush strength and standard deviation calculated. These results are given in Table 3 and illustrated in Fig. 1.

**Table 3:**

| Example | Total Pore volume (mL/g) | (Average flat plate crush strength± std. dev.) (lb/mm) |
|---|---|---|
| 19 | 0.816 | 2.601 ± 0.265 (1.18 ± 0.12 kg/mm) |
| 20 | 0.798 | 3.095 ± 0.584 (1.404 ± 0.265 kg/mm) |
| 21 | 1.090 | 1.624 ± 0.265 (0.737 ± 0.12 kg/mm) |
| 22 | 1.035 | 1.798 ± 0.287 (0.816 ± 0.13 kg/mm) |

### Example Nos. 23 and 24

For Example Nos. 23 and 24, the surface composition of randomly selected commercially available porous shaped bodies of alpha-alumina is determined by XPS.

Example No. 23: Porous shaped bodies of alpha alumina CS303 (United Catalyst Inc, Louisville, KY, USA, now know as Sud-Chemie) are loaded into the reactor, and heated under a vacuum to 950°C. SiF₄ gas is then added to the reactor until a pressure of 300 torr (40 kPa) is achieved; the reactor is then closed and the shaped porous bodies allowed to contact the SiF₄ gas for 90 min at 950°C. The gas is then removed from the reactor and the bodies cool to room temperature. The surface composition of randomly chosen pellets after treatment is determined by XPS and set forth in Table 4.

In Example No. 24, porous shaped bodies of alpha alumina (Norton SA-5473 (Lot 07-91039 12-20 mesh) (Saint-Gobain-Norpro (Stow OH USA)), are loaded into the reactor, and heated under a vacuum to 950°C. SiF₄ gas is then added to the reactor until a pressure of 300 torr (40 k P a) is achieved; the reactor is then closed and the porous shaped bodies allowed to contact the SiF₄ gas for 90 min at 950°C. The gas is then removed from the reactor and the bodies cool to room temperature. The surface composition of randomly chosen pellets after treatment is determined by XPS and set forth in Table 4.

**Table 4:**

| | Atomic% | | | | | | | | |
|---|---|---|---|---|---|---|---|---|---|
| Example | 0 | | C | Al | Si | Na | F | Ti | Ca |
| 23 before treatment | 59.8 | | 6.0 | 29.4 | 0.6 | 3.4 | nd | nd | 0.3 |
| 23 after treatment | 58.6 | | 2.6 | 26.6 | 5.2 | 1.6 | 3.9 | 0.2 | 0.7 |
| 24 before treatment | 59.9 | | 7.0 | 29.6 | 0.3 | nd | nd | 2.7 | nd |
| 24 after treatment | 57.9 | | 3.9 | 28.6 | 2.6 | 1.3 | 3.3 | 0.9 | 0.1 |

| | | | | | | | | | |
|---|---|---|---|---|---|---|---|---|---|
| nd=not detected | | | | | | | | | |

### Example Nos. 25 through 29

For Example Nos. 25 through 29, a precursor composition is calcined and then contacted with SiF₄ gas as shown in Table 5. For Example Nos. 25, 26, 28 and 29, the samples are prepared by mixing a 50g batch of the alumina and additive component in 30g of an 8% aqueous methylcellulose (Methocel^{®} A15LV) solution with a mechanical stirrer, drying the mix at room temperature, crushing the dried mix and then dry pressing into about 1" (2.5 cm) disks approximately 2 mm thick. Fused silica is obtained from Harbison-Walker (ANH Refractories, Moon Township, PA USA), manganese oxide (Sigma-Aldrich, St. Louis, MO USA), granular zirconium silicate in powder form with a median particle size of about 120 microns, and magnesium silicate (Magnesol, The Dallas Group of America, Jefferson, IN USA). The dry pressed disks are then calcined and contacted with the SiF₄ gas. For Example No. 27, 1/4" (0,64 cm) rings are extruded of Versal V-250, calcined for 2 hours at 750°C, then impregnated to the point of incipient wetness with a 0.166 molar solution of magnesium nitrate. The magnesium nitrate solution is prepared by dissolving magnesium nitrate hexahydrate (Fisher, USA) in water. After impregnation, the extrudates are dried, then calcined and contacted with the SiF₄ gas. Samples are examined by SEM microscopy which shows that the product is primarily interlocked platelets. X-ray diffraction (XRD) analysis of the product show a variety of crystalline products which are given in Table 5.

**Table 5:**

| Example | Precursor Composition | Calcination Temp (°C) | SiF₄ Contact Temp (°C) | SiF₄ Pressure (torr) ** | Contact Time (min) | XRD Result (PDF Card No. and Phase) * |
|---|---|---|---|---|---|---|
| 25 | 97.0% Selecto alumina (κ-alumina); 3.0% SiO₂ (fused silica) | 1000 | 720 to 1120 | 600 torr from 720C to 850C; 500 torr from 850C to 1120C | 410 | 10-0173 Corundum |
| | | | | | | 82-1218 Topaz |
| | | | | | | 15-0776 Mullite |
| 26 | 95.0% Selecto alumina (κ-alumina); 3.0% Si02; 2.0%Mg0 | 1000 | 720 to 1120 | 600 torr from 720C to 850C; 500 torr from 850C to 1120C | 410 | 10-0173 Corundum |
| | | | | | | 82-1218 Topaz |
| | | | | | | 15-0776 Mullite |
| | | | | | | 72-2231 Magnesium fluoride |
| 27 | 97.7% calcined Versal V-250 (y-alumina); 2.3% Mg (NO₃)₂ | 750 | 925 | 80 | 120 | 10-0173 Corundum |
| | | | | | | 72-2231 Magnesium fluoride |
| | | | | | | 27-0605 Cristobalite |
| 28 | 95.0% Alphabond 300; 5.0% Zirconium silicate | 750 | 930 | 100 | 90 | 10-0173 Corundum |
| | | | | | | 06-0266 Zircon |
| | | | | | | 27-0605 Cristobalite |
| 29 | 95.0% Alphabond 300; 5.0% Magnesium Silicate | 750 | 930 | 100 | 90 | 10-0173 Corundum |
| | | | | | | 71-2401 Norbergite |
| | | | | | | 15-0776 Mullite |
| | | | | | | 27-0605 Cristobalite |

| | | | | | | |
|---|---|---|---|---|---|---|
| *PDF refers to Powder Diffraction File^{™} which is a database complied by the International Centre for Diffraction Data in Newtown Square, Pennsylvania, USA. **80 torr = 10.7 kPa; 100 torr = 13.3 kPa; 500 torr = 66.7 kPa; 600 torr = 80 kPa. | | | | | | |

The methods of preparation of Carriers No. 1 through 8 are given in previous examples. Carrier 1 is described in Example No. 8. Carriers 2 through 5 are the carriers described in Example No. 14, Carrier 6 is described in Example No. 15, Carrier 7 is described in Example No. 4 and Carrier 8 is described in Example No. 5. The carrier described in Example No. 14 (Carrier 2 through 5) is divided and heat-treated at various temperatures before being made into a catalyst. Characteristics of Carrier Nos. 1 through 8 are set forth in Table 6, below.

**Table 6: Carriers Used to Prepare Ethylene Oxide Catalysts**

| Carrier No. | Surface Area (m²/g) | Median Pore Diameter (microns) | Total pore volume (mL/g) | Heat Treatment Temp. (°C) | Example No. |
|---|---|---|---|---|---|
| 1 | 1.56 | 2.2 | 0.67 | None | 8 |
| 2 | 2.60 | 0.8 | 0.87 | None | 14 |
| 3 | 2.61 | 1.1 | 0.93 | 900 | 14 |
| 4 | 2.50 | 0.9 | 0.95 | 1100 | 14 |
| 5 | 2.37 | 1.0 | 0.93 | 1300 | 14 |
| 6 | 1.65 | 2.3 | 0.97 | 1300 | 15 |
| 7 | 1.40 | 2.6 | 1.00 | None | 4 |
| 8 | 0.90 | - | 0.78 | None | 5 |

The surface area of each carrier is determined by B.E.T. using nitrogen. The total pore volume and median pore diameter of the carriers are determined by mercury porosimetry to a maximum pressure of 60,000 psig (413.8 MPa). Carriers 2, 3, 4 and 5 are portions of the carrier prepared in Example No. 14 which are heated separately in open crucibles for 2 hours at temperatures of 900°C, 1100°c and 1300°C, respectively, after their conversion to alpha-alumina and before catalyst preparation.

### Catalyst Preparation

A silver-amine-oxalate impregnation solution is obtained from the Union Carbide Corporation EO/EG catalyst unit of The Dow Chemical Company, which contains about 30 percent silver oxide, 18 percent oxalic acid, 17 percent ethylenediamine, 6 percent monoethanolamine, and 27 percent distilled water by weight, and a procedure to make the solution by (1) mixing 1.14 parts of ethylenediamine (high purity grade) with 1.75 parts of distilled water; (2) slowly adding 1.16 parts of oxalic acid dihydrate (reagent grade) to the aqueous ethylenediamine solution such that the temperature of the solution did not exceed 40°C, (3) slowly adding 1.98 parts of silver oxide, and (4) adding 0.40 parts of monoethanolamine (Fe and Cl free). The carriers shown in Table 5 are vacuum impregnated with the impregnation· silver solution described above.

The carrier is impregnated in an appropriately sized glass or stainless steel cylindrical vessel which is equipped with suitable stopcocks for impregnating the carrier under vacuum. A suitable separatory funnel which is used for containing the impregnating solution is inserted through a rubber stopper into the top of the impregnating vessel. The impregnating vessel containing the carrier is evacuated to approximately 1-2" mercury absolute (3.39-6.77 kPa) for 10 to 30 minutes, after which the impregnating solution is slowly added to the carrier by opening the stopcock between the separatory funnel and the impregnating vessel. After all the solution empties into the impregnating vessel (~15 seconds), the vacuum is released and the pressure is returned to atmospheric. Following addition of the solution, the carrier remains immersed in the impregnating solution at ambient conditions for 5 to 30 minutes, and is thereafter drained of excess solution for 10 to 30 minutes.

The silver-impregnated carrier is then roasted as follows to effect reduction of silver on the catalyst surface. The impregnated carrier is spread out in a single layer on stainless steel wire mesh trays then placed on a stainless steel belt (spiral weave) and transported through a 2" x 2" (5.1 cm x 5.1 cm) square heating zone for 2.5 minutes, or equivalent conditions are used for a larger belt operation. The heating zone is maintained at 500°C by passing hot air upward through the belt and about the catalyst particles at the rate of 266 standard cubic feet per hour (SCFH) (7.53 cubic metres per hour). After being roasted in the heating zone, the catalyst is cooled in the open air to room temperature and weighed.

Next, the silver-impregnated carrier is vacuum impregnated with a second silver impregnation solution containing both the silver oxalate amine solution and the catalyst promoters. The second impregnation solution is composed of all of the drained solution from the first impregnation plus a fresh aliquot of the first solution, or a new solution is used. The promoters, in either aqueous solution or neat form, are added (in the ascending numeric order listed in Table 7) with stirring. In Catalysts A through F and H, two molar equivalents of diammonium ethylenediaminetetraacetic acid (EDTA) are added with the manganese promoter in order to stabilize the manganese in the impregnation solution. In Catalyst G, one excess molar equivalent of diammonium EDTA is added for the same purpose.

The impregnation, draining and roasting steps for this second impregnation are carried out analogously to the first impregnation.

The twice-impregnated carrier, i.e., the finished catalyst, is again weighed, and based upon the weight gain of the carrier in the second impregnation, the weight percent of silver and the concentration of the promoters are calculated (results given in Table 7). The finished catalyst is then employed in an ethylene epoxidation reaction, the results of which are given in Example No. 31.

**Table 7: Catalyst Preparations**

| Catalyst No. | A | B | C | D |
|---|---|---|---|---|
| Carrier No. | 1 | 2 | 3 | 4 |
| Promoter 1 | CsOH | CsOH | CsOH | CsOH |
| Promoter 2 | Cs₂SO₄ | Cs₂SO₄ | Cs₂SO₄ | Cs₂SO₄ |
| Promoter 3 | Mn(NO₃)₂ | Mn(NO₃)₂ | Mn(NO₃)₂ | Mn(NO₃)₂ |
| Chelating Agent | (NH₄)₂H₂(EDTA) | (NH₄)₂H₂(EDTA) | (NH₄)₂H₂(EDTA) | (NH₄)₂H₂(EDTA) |
| Total Wt. percent Silver | 33.7 | 41.6 | 41.5 | 41.7 |
| Promoter 1; ppm | 953 Cs | 980 Cs | 993 Cs | 972Cs |
| Promoter 2; ppm | 268 S04 | 274 SO4 | 278 SO4 | 272 S04 |
| Promoter 3; ppm | 172 Mn | 177 Mn | 179 Mn | 175 Mn |

**Table 7: Catalyst Preparations, continued**

| Catalyst No. | E | F | G | H |
|---|---|---|---|---|
| Carrier No. | 5 | 6 | 7 | 8 |
| Promoter 1 | CsOH | CsOH | KNO₃ | (NH₄)₂SO₄ |
| Promoter 2 | Cs₂SO₄ | Cs₂SO₄ | K₂Mn(EDTA) | CsOH |
| Promoter 3 | Mn(NO₃)₂ | Mn(NO₃)₂ | | Mn(NO₃)₂ |
| Promoter 4 | | | | (NH₄)₂ReO₄ |
| Chelating Agent | (NH₄)₂H₂(EDTA) | (NH₄)₂H₂(EDTA) | (NH₄)₂H₂(EDTA) | (NH₄)₂H₂(EDTA) |
| Total Wt. percent Silver | 41.4 | 41.3 | 44.5 | 37.2 |
| Promoter 1; ppm | 987 Cs | 839 Cs | 1735 K | 168 S04 |
| Promoter 2; ppm | 276 SO4 | 237 SO4 | 153 Mn | 649 Cs |
| Promoter 3; ppm | 178 Mn | 154 Mn | | 60Mn |
| Promoter 4; ppm | | | | 276 Re |

### Example No. 31

A standard back-mixed autoclave with internal gas recycle or a single-pass tubular reactor is used for catalyst testing. There is some variation in ethylene, oxygen and gas phase modifier/promoter feed concentrations which are given in Table 8. Well known, back-mixed, bottom-agitated autoclaves similar to those described in FIG. 2 of the paper by J. M. Berty entitled "Reactor for Vapor Phase-Catalytic Studies," in Chemical Engineering Progress, Vol. 70, No.5, pages 78-84, 1974, are used as one of the reactors. The inlet conditions include the following:

**Table 8: Ethylene Epoxidation Process Conditions**

| Component | Oxygen Process Conditions-I Mole percent | Oxygen Process Conditions-II Mole percent | Oxygen Process Conditions-III Mole percent |
|---|---|---|---|
| Ethylene | 30.0 | 30.0 | 30.0 |
| Oxygen | 8.0 | 8.0 | 8.0 |
| Ethane | 0.5 | 0.0 | 0.5 |
| Carbon Dioxide | 6.5 | 0.0 | 3.0 |
| Nitrogen | Balance of gas | Balance of gas | Balance of gas |
| Parts per million Ethyl Chloride | Optimum for Efficiency | Optimum for Efficiency | Optimum for Efficiency |
| Parts per million Nitric Oxide | None | Optimum for Efficiency | None |
| Type of Reactor | Tube | CSTR | Tube |
| Amount of Catalyst | 0.5 g | 40cm³ | 0.5 g |
| Total Outlet Flow Rate | 180 cm³/min | 11.3 SCFH (0.32 m³/h) | 180 cm³/min |

The catalyst test procedure used for the tubular reactor in the Ethylene Epoxidation Process Conditions is the following: Approximately 5g of catalyst is crushed with a mortar and pestle, then sieved to 30/50 U.S. Standard mesh. From the meshed material, 0.5 g. is charged to the microreactor made of 0.25 inch (0,64 cm) O.D. stainless steel (wall thickness 0.035 inches (0.89 mm)). Glass wool is used to hold the catalyst in place. The reactor tube is fitted into a heated brass block which has a thermocouple placed against it. The block is enclosed in an insulated box. Feed gas is passed over the heated catalyst at a pressure of 200 psig (1.5 MPa). The reactor flow is adjusted and recorded at standard pressure and room temperature.

The catalyst test procedure used for autoclaves in the Ethylene Epoxidation Process Conditions is as follows: 40 cc of catalyst is charged to the back-mixed autoclave and the weight of the catalyst noted. The back-mixed autoclave is heated to about reaction temperature in a nitrogen flow of about 10 SCFH (0.28 m³/h)with the fan operating at 1500 rpm.
SCFH refers to cubic feet per hour at standard temperature and pressure, namely, 0°C. and one atmosphere (101.3 kPa). The nitrogen flow is then discontinued and the above-described feed stream introduced into the reactor. The total gas outlet flow is adjusted to 11.3 SCFH (0.32 m³/h) and the total pressure maintained constant at 275 psig (2 MPa). The temperature is adjusted over the next few hours to provide the desired outlet ethylene oxide. The optimum efficiency may be obtained by adjusting ethyl chloride and/or nitric oxide. Temperature (°C) or outlet epoxide as a function of temperature and catalyst efficiency are typically obtained as the responses describing the catalyst performance. The catalyst performance is monitored carefully to make sure the catalyst performance has reached steady state conditions. Ethyl chloride and nitric oxide concentrations may be adjusted further to maintain maximum efficiency. In catalyst aging tests, the efficiency of the catalyst to ethylene epoxide and the rate of deactivation (temperature rise and / or efficiency loss) are obtained.

The standard deviation of a single test result reporting catalyst efficiency in accordance with the procedures described above is about 0.5 percent efficiency units for both test units. The typical standard deviation of a single test result reporting catalyst activity in accordance with the procedure described above is about 2°C or 0.1% mole EO. The standard deviation, of course, will depend upon the quality of the equipment and precision of the techniques used in conducting the tests, and thus will vary. The test results reported herein are believed to be within the standard deviation set forth above.

For Catalysts A-F, Table 9 shows the efficiency and temperature required to produce 1 mole percent of ethylene oxide from Catalysts A through F (Table 7) under the conditions described in Table 8 as Oxygen Process Conditions -I. Some results are interpolated in order to obtain the temperature and efficiency for exactly 1.00 mole percent outlet ethylene oxide.

**Table 9: Catalysts A-F**

| Catalyst | Carrier | Alumina Source | Heat Treatment Temp (°C) | BET Surface Area (m²/g) | Total Pore Volume (mL/g) | Median Pore Dia. (µm) | Eff@ 1.0%EO (%) | Temp@ 1.0%EO (°C) |
|---|---|---|---|---|---|---|---|---|
| A | 1 | Catapal B | none | 1.86 | 0.68 | 2.22 | 82.8 | 228 |
| B | 2 | Alphabond 300 | none | 2.60 | 0.87 | 0.78 | 83.3 | 220 |
| C | 3 | Alphabond 300 | 900 | 2.61 | 0.93 | 1.12 | 83.5 | 219 |
| D | 4 | Alphabond 300 | 1100 | 2.50 | 0.95 | 0.93 | 83.8 | 222 |
| E | 5 | Alphabond 300 | 1300 | 2.37 | 0.93 | 0.97 | 83.7 | 218 |
| F | 6 | 80%Alphabond | 1300 | 1.65 | 0.975 | 2.32 | 82.0 | 232 |
| | | 20% Catapal B | | | | | | |

For Catalyst G, Table 10 gives the amount of ethylene oxide in mole percent and efficiency (i.e., the selectivity of the reaction of ethylene to ethylene oxide) of the catalyst at various reactor temperatures under Ethylene Oxide Process Conditions -Oxygen Process Conditions II (Table 8). Data are collected during the first week of catalyst testing.

**Table 10: Catalyst G**

| Temperature (°C) | Outlet EO (mole%) | Efficiency (%) | Inlet EC1 (ppm) | Inlet NO (ppm) |
|---|---|---|---|---|
| 220 | 1.19 | 91.3 | 3.0 | 2 |
| 230 | 1.66 | 90.6 | 3.5 | 3 |
| 235 | 1.92 | 90.2 | 4.0 | 4 |
| 240 | 2.19 | 90.0 | 5.0 | 5 |

For Catalyst H, the amount of ethylene oxide in mole percent and efficiency (i.e., the selectivity of the reaction of ethylene to ethylene oxide) of the catalyst at a reactor temperature of 240°C at two different inlet ethyl chloride (ECl) levels and under Ethylene Oxide Process Conditions- Oxygen Process Conditions III (Table 8) are given in Table 11.

**Table 11: Catalyst H**

| Temperature (°C) | Outlet EO (mole%) | Efficiency (%) | Inlet EC1 (ppm) |
|---|---|---|---|
| 240 | 0.99 | 85.8 | 4 |
| | 0.95 | 86.5 | 5 |

### Example Nos. 32 and 33

For Example Nos. 32 and 33, precursor bodies having the shape of about 1/4" (0.64 cm) O.D. (3/32" (0.24cm)I.D.) 1/4" (0.64 cm) long rings are prepared as follows. An extrudable paste is prepared by mix- mulling 1kg of Versal V-250, a pseudo-boehmite, (UOP LLC, DesPlaines, IL USA), 65g of Methocel^{®} A4M (Dow Chemical Company, Midland MI USA), 850g of water, 20g of oleic acid (VWR Scientific Products, West Chester PA USA) in a stainless steel muller for 12 min. The paste is aged for 24h in a sealed container, and then extruded with an 18 mm counter rotating twin-screw extruder equipped with a chopping mechanism at the die outlet. The resulting ring-shaped extrudate pellets are dried for 36 to 72 hours at 60°C in flowing air, then slowly heated to 1000°C and held for 2 hours at 1000°C to remove the organic components and to dehydrate the Versal V-250.

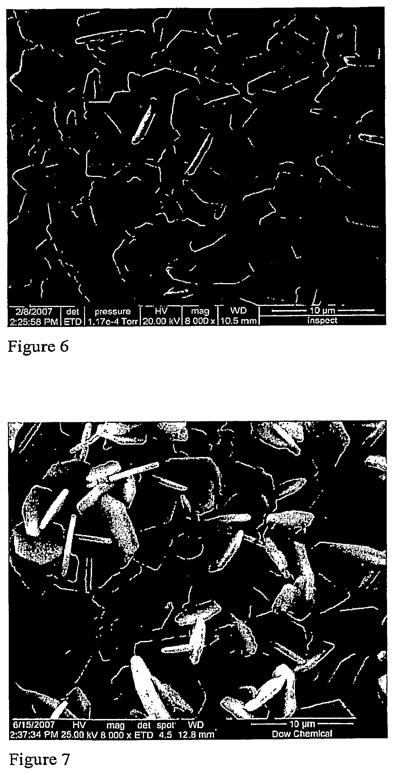

In Example No. 32, the precursor bodies are loaded into a controlled atmosphere reactor employing a 6" (15.2 cm) I.D. alumina tube instead of a 6" (15.2 cm) ID. quartz tube and heated under a vacuum to 810°C. HFC-134a gas is then added to the reactor until a pressure of 300 torr (40 kPa) is achieved. The reactor is then closed and the precursor bodies are allowed to contact the gas for 60 min at 810°C. Then the reactor is heated at 2°C/min until a temperature of 930°C is reached. When the reactor temperature reaches 930°C, the gas is removed from the reactor and the reactor and its contents are allowed to cool to room temperature. Immediately after the gas is removed from the reactor, N2 gas is added to a pressure of 400 torr (53.3 kPa), then removed. This step is repeated twice to facilitate the removal of fluorine-containing gas from the system. SEM analysis of randomly chosen rings show the product is primarily interlocked platelets. See Fig. 6. The surface composition of randomly chosen pellets is determined by XPS and the results are given in Table 12. The flat plate crush strength of 10 randomly selected pellets is measured and the average flat plate crush strength is 1.207 lbs/mm (0.547 kg/mm) with standard deviation of 0.300 lbs/mm (0.136 kg/mm).

Using the support prepared in Example No. 32, a catalyst containing calculated promoter and silver levels of 1666 ppm K, 147 ppm Mn and 37.4 wt% Ag is prepared using potassium nitrate and K₂Mn(EDTA) and (NKU)₂H₂(EDTA) solutions via the same methods described earlier. A thirty gram sample of this catalyst is tested in a Rotoberty reactor under Oxygen Process Conditions II shown in Table 8. The inlet feeds are 8% oxygen, 30% ethylene, 5 ppm ethyl chloride and 5 ppm nitric oxide and the initial reactor temperature is 220°C. On the second day, the temperature is increased to 230°C and the reactor undergoes an automatic shutdown. The reactor is restarted at 240°C on the third day. On the fourth day temperature is increased to 255°C. On the fifth and sixth day, the temperature is set at 240°C. On the fifth day, the catalyst produced 1.75% ethylene oxide with a selectivity of 87.5%.

In Example No. 33, a portion of the product of Example 32 is placed in an alumina crucible and heated in a box furnace at about 4°C/min to 1400°C, held at 1400°C for 4 hours, then cooled to room temperature. The surface composition of randomly chosen pellets is determined by XPS and the results are given in Table 12. The flat plate crush strength of 10 randomly selected pellets is measured and the average flat plate crush strength is 2.553 lbs/mm (1.158 kg/mm) with standard deviation of 0.542 lbs/mm (0,246 kg/mm).

Using the support prepared in Example No. 33, a catalyst containing calculated promoter and silver levels of 1736 ppm K, 151 ppm Mn and 36.9 wt% Ag is prepared using potassium nitrate and K₂Mn(EDTA) and (NH₄)₂H₂(EDTA) solutions via the same methods described earlier. A thirty gram sample of this catalyst is tested in a Rotoberty reactor under Oxygen Process Conditions II shown in Table 8. The inlet feeds are 8% oxygen, 30% ethylene, 5 ppm ethyl chloride and 5 ppm nitric oxide and the initial reactor temperature is 220°C. On the second day, the temperature is increased to 230°C. The reactor is restarted at 240°C on the third day. On the fourth day temperature is increased to 255°C. On the fifth and sixth day, the temperature is set at 240°C. On the fifth day, the catalyst produced 1.53% ethylene oxide with a selectivity of 86.4%.

**Table 12:**

| | Atomic% | | | | | | |
|---|---|---|---|---|---|---|---|
| Example | O | C | Al | Si | F | Na | B |
| 32 | 47.4 | 9.7 | 30.7 | nd | 10.9 | 0.5 | nd |
| 33 | 54.1 | 14.9 | 28.1 | 0.7 | 0.9 | nd | nd |

| | | | | | | | |
|---|---|---|---|---|---|---|---|
| nd = not detected | | | | | | | |

### Example Nos. 34 and 35

For Example Nos. 34 and 35, the surface composition of randomly selected commercially available porous shaped bodies of alpha-alumina is determined by XPS.

Example No. 34: Porous shaped bodies of alpha alumina 06590-S H218C-6 HT (United Catalyst Inc, Louisville, KY, USA, now known as Sud-Chemie) are loaded into the reactor employing a 6" (15.2 cm) I.D. alumina tube instead of the 6" (15.2 cm) I.D. quartz tube, and heated under a vacuum to 840°C. HFC-134a gas is then added to the reactor until a pressure of 300 torr (40 kPa) is achieved. The reactor is then closed and the precursor bodies are allowed to contact the gas for 180 min at 840°C. Then the reactor is heated at 2°C/min. When a temperature of 900°C is reached, the gas is removed from the reactor. N₂ gas is added to a pressure of 400 torr (53.3 kPa) and then removed. Additional N₂ gas is added to a pressure of 400 torr (53.3 kPa) and then removed. The reactor is then maintained under a dynamic vacuum until a temperature of 980°C is reached. When the reactor temperature reaches 980°C, the reactor and its contents are allowed to cool to room temperature. The surface composition of randomly chosen pellets after treatment is determined by XPS and set forth in Table 13.

In Example No. 35, porous shaped bodies of alpha alumina (Norton SA-5502 (1/4" rings) (St. Gobain-Norpro (Stow OH USA)), are loaded into the reactor employing a 6" (15.2 cm) I.D. alumina tube instead of the 6" (15.2 cm) ID. quartz tube, and heated under a vacuum to 840°C. HFC-134a gas is then added to the reactor until a pressure of 300 torr ( 4 0 k P a) is achieved. The reactor is then closed and the precursor bodies are allowed to contact the gas for 180 min at 840°C. Then the reactor is heated at 2°C/min. When a temperature of 900°C is reached, the gas is removed from the reactor. N₂ gas is added to a pressure of 400 torr ( 5 3 . 3 k P a) and then removed. Additional N₂ gas is added to a pressure of 400 torr (53.3 k P a) and then removed. The reactor is then maintained under a dynamic vacuum until a temperature of 980°C is reached. When the reactor temperature reaches 980°C, the reactor and its contents are allowed to cool to room temperature. The surface composition of randomly chosen pellets after treatment is determined by XPS and set forth in Table 13.

**Table 13:**

| | Atomic% | | | | | | | | |
|---|---|---|---|---|---|---|---|---|---|
| Example | O | | C | Al | Si | Na | F | Ti | Cl |
| 34 before treatment | 52.9 | | 10.8 | 30.8 | nd | 1.1 | 3.4 | 0.9 | nd |
| 34 after treatment | 40.4 | | 22.0 | 28.1 | nd | 0.3 | 9.0 | nd | nd |
| 35 before treatment | 50.6 | | 9.6 | 29.4 | 1.2 | 3.6 | 5.0 | nd | nd |
| 35 after treatment | 41.8 | | 16.0 | 29.8 | nd | 0.3 | 11.0 | nd | nd |

| | | | | | | | | | |
|---|---|---|---|---|---|---|---|---|---|
| nd=not detected | | | | | | | | | |

### Example No. 36 and 37

For Example Nos. 36 and 37, precursor bodies having the shape of about 1/4" (0.64 cm) O.D. (3/32"(0.24 cm) ID.) 1/4" (0.64 cm) long rings are prepared as described for Examples 32 and 33.

In both Examples Nos. 36 and 37, the precursor bodies are loaded into a controlled atmosphere reactor based on a Series 3700 Model 12X12X24 graphite vacuum furnace.

In Example No. 36, the precursor bodies are heated under a vacuum to 800°C. HFC-134a gas is then added to the reactor until a pressure of 150 torr (20 k P a) is achieved. The reactor is then closed and the precursor bodies are allowed to contact the gas for 120 min at 820°C. Then the reactor is heated at 2°C/min until a temperature of 950°C is reached. When the reactor temperature reaches 950°C, the gas is evacuated and a series of seven N₂ purges is initiated. The reactor temperature is held at 950°C for 120 min before the reactor and its contents are allowed to cool to room temperature. SEM analysis of randomly chosen rings show the product is primarily interlocked platelets. See Fig. 7.

In Example No. 37, the precursor bodies are heated under a vacuum to 820°C. HFC-134a gas is then added to the reactor until a pressure of 150 torr (20 kPa) is achieved. The reactor is then closed and the precursor bodies are allowed to contact the gas for 120 min at 820°C. At this time, a series of eleven N₂ purges is initiated. One hour later the reactor is heated at 2°C/min until a temperature of 900°C is reached. The reactor temperature is held at 900°C for 2 h, then the reactor is heated at 5°C/min until a temperature of 1400°C is reached. The reactor temperature is held at 1400°C for 4 h before the reactor and its contents are allowed to cool to room temperature. SEM analysis of randomly chosen rings show the product is primarily interlocked platelets.

The use of the terms "a" and "an" and "the" and similar referents in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Of course, variations of those preferred embodiments will become apparent to those of ordinary skill in the art upon the foregoing description.

## Claims

1. A method for modifying the surface composition of alpha-alumina to cause the surface composition of the alpha-alumina to contain elements other than aluminum, fluorine and oxygen, the method comprising:
a. providing a vessel in which a total pressure can be varied and controlled;
b. introducing into the vessel alpha-alumina having a surface composition;
c. introducing into the vessel, as a fluorine-containing gas at the conditions of introduction, a fluorine-containing compound selected from the group consisting of SiF₄, BF₃, CF₄, CHF₃, C₂H₂F₄ and mixtures of two or more of the gases HF, SiF₄, BF₃, CF₄, CHF₃ and C₂H₂F₄; and
d. contacting the introduced fluorine-containing compound with at least a portion of the alpha-alumina for one or more periods of time, at a total pressure ranging from 1 to 100,000 torr (133 Pa to 13.3 MPa),_and at temperatures between 750 and 1200°C to modify the surface composition of the alpha-alumina.

2. The method according to claim 1, wherein the alpha alumina of step b. is prepared by:
i. providing a vessel in which a total pressure can be varied and controlled;
ii. introducing into the vessel a shaped precursor body which comprises at least one alpha-alumina precursor;
iii. heating the vessel to a temperature of from 750 to 1150°C;
iv. after heating the vessel, introducing into the vessel, as a fluorine-containing gas at the conditions of introduction, a fluorine-containing compound selected from the group consisting of HF, SiF₄, BF₃, CF₄, CHF₃, C₂H₂F₄ and mixtures of two or more of these gases;
v. establishing a total pressure within the vessel of between about 1 torr and 100,000 torr (133 Pa to 13.3 MPa),
vi. contacting the introduced fluorine-containing compound with at least a portion of the shaped precursor body, for a time of 1 minute to 48 hours at a temperature of from 750 to 1150°C and a partial pressure of from l to 10,000 torr(133 Pato 1.33 MPa), sufficient to convert at least 50% of the alpha-alumina precursor to alpha-alumina platelets.

## Patentansprüche

1. Verfahren zum Modifizieren der Oberflächenzusammensetzung von Alpha-Aluminiumoxid, um zu bewirken, dass die Oberflächenzusammensetzung des Alpha-Aluminiumoxids andere Elemente als Aluminium, Fluor und Sauerstoff enthält, das Verfahren umfassend:
a. Bereitstellen eines Behälters, in dem ein Gesamtdruck variiert und gesteuert werden kann;
b. Einführen in den Behälter von Alpha-Aluminiumoxid, das eine Oberflächenzusammensetzung aufweist;
c. Einführen in den Behälter, als ein fluorhaltiges Gas bei den Bedingungen einer Einführung, einer fluorhaltigen Verbindung, ausgewählt aus der Gruppe, bestehend aus SiF₄, BF₃, CF₄, CHF₃, C₂H₂F₄ und Mischungen von zwei oder mehr der Gase HF, SiF₄, BF₃, CF₄, CHF₃ und C₂H₂F₄; und
d. Inberührungbringen der eingeführten fluorhaltigen Verbindung mit mindestens einem Anteil des Alpha-Aluminiumoxids für einen oder mehrere Zeiträume bei einem Gesamtdruck in einem Bereich von 1 bis 100.000 Torr (133 Pa bis 13,3 MPa)_und bei Temperaturen zwischen 750 und 1200 °C, um die Oberflächenzusammensetzung des Alpha-Aluminiumoxids zu modifizieren.

2. Verfahren nach Anspruch 1, wobei das Alpha-Aluminiumoxid aus Schritt b. hergestellt wird durch:
i. Bereitstellen eines Behälters, in dem ein Gesamtdruck variiert und gesteuert werden kann;
ii. Einführen in den Behälter eines geformten Vorläuferkörpers, der mindestens einen Alpha-Aluminiumoxid-Vorläufer umfasst;
iii. Erhitzen des Behälters auf eine Temperatur von 750 bis 1150 °C;
iv. nach dem Erhitzen des Behälters, Einführen, als ein fluorhaltiges Gas bei den Bedingungen der Einführung, einer fluorhaltigen Verbindung, ausgewählt aus der Gruppe, bestehend aus HF, SiF₄, BF₃, CF₄, CHF₃, C₂H₂F₄ und Mischungen von zwei oder mehr dieser Gase;
v. Einstellen eines Gesamtdrucks innerhalb des Behälters von zwischen etwa 1 Torr und 100.000 Torr (133 Pa bis 13,3 MPa),
vi. Inberührungbringen der eingeführten fluorhaltigen Verbindung mit mindestens einem Anteil des geformten Vorläuferkörpers für eine Zeit von 1 Minute bis 48 Stunden bei einer Temperatur von 750 bis 1150 °C und einem Partialdruck von 1 bis 10.000 Torr (133 Pa bis 1,33 MPa), ausreichend, um mindestens 50 % des Alpha-Aluminiumoxid-Vorläufers in Alpha-Aluminiumoxid-Plättchen umzuwandeln.

## Revendications

1. Procédé de modification de la composition de surface de l'alpha-alumine pour amener la composition de surface de l'alpha-alumine à contenir des éléments autres que l'aluminium, le fluor et l'oxygène, le procédé comprenant :
a. la fourniture d'un récipient dans lequel une pression totale peut être variée et commandée ;
b. l'introduction dans le récipient d'alpha-alumine ayant une composition de surface ;
c. l'introduction dans le récipient, en tant que gaz contenant du fluor aux conditions d'introduction, d'un composé contenant du fluor choisi dans le groupe constitué de SiF₄, BF₃, CF₄, CHF₃, C₂H₂F₄ et des mélanges de deux ou plusieurs des gaz HF, SiF₄, BF₃, CF₄, CHF₃ et C₂H₂F₄ ; et
d. la mise en contact du composé contenant du fluor introduit avec au moins une partie de l'alpha-alumine pendant un ou plusieurs laps de temps, à une pression totale allant de 1 à 100 000 torr (133 Pato, 13,3 Mpa), et à des températures comprises entre 750 et 1 200 °C, pour modifier la composition de surface de l'alpha-alumine.

2. Procédé selon la revendication 1, dans lequel l'alumine alpha de l'étape b. est préparée par :
i. la fourniture d'un récipient dans lequel une pression totale peut être variée et commandée ;
ii. l'introduction dans le récipient d'un corps de précurseur façonné qui comprend au moins un précurseur d'alpha-alumine ;
iii. le durcissement du mélange à une température allant de 750 à 1150 °C ;
iv. après chauffage de la cuve, l'introduction dans le récipient, en tant que gaz contenant du fluor aux conditions d'introduction, un composé contenant du fluor choisi dans le groupe constitué de HF, SiF₄, BF₃, CF₄, CHF₃, C₂H₂F₄ et des mélanges de deux ou plusieurs de ces gaz ;
v. l'établissement d'une pression totale à l'intérieur du récipient comprise entre environ 1 torr et 100 000 torr (133 Pa à 13,3 MPa),
vi. la mise en contact du composé contenant du fluor introduit avec au moins une partie du corps de précurseur façonné, pendant une durée de 1 minute à 48 heures à une température allant de 750 à 1150 °C et une pression partielle allant de 1 à 10 000 torr (133 Pato 1,33 MPa), suffisante pour convertir au moins 50 % du précurseur d'alpha-alumine en plaquettes d'alpha-alumine.
